(19) Europäisches Patentamt
European Patent Office
Office européen des brevets

(11) **EP 3 559 953 B1**

(12) **EUROPÄISCHE PATENTSCHRIFT**

(45) Veröffentlichungstag und Bekanntmachung des Hinweises auf die Patenterteilung:
**10.05.2023   Patentblatt 2023/19**

(21) Anmeldenummer: **17822205.5**

(22) Anmeldetag: **12.12.2017**

(51) Internationale Patentklassifikation (IPC):
**G16H 40/67** (2018.01)   **G06T 7/73** (2017.01)

(52) Gemeinsame Patentklassifikation (CPC):
**G16H 40/67; G06T 7/74**

(86) Internationale Anmeldenummer:
**PCT/EP2017/082340**

(87) Internationale Veröffentlichungsnummer:
**WO 2018/114454 (28.06.2018 Gazette 2018/26)**

(54) **VORRICHTUNG, VERFAHREN UND COMPUTERPROGRAMM ZUR ERFASSUNG VON OPTISCHEN BILDDATEN UND ZUR BESTIMMUNG EINER LAGE EINER SEITENBEGRENZUNG EINER PATIENTENLAGERUNGSVORRICHTUNG**

DEVICE, METHOD AND COMPUTER PROGRAM FOR CAPTURING OPTICAL IMAGE DATA AND FOR DETERMINING A POSITION OF A LATERAL BOUNDARY OF A PATIENT SUPPORTING DEVICE

DISPOSITIF, PROCÉDÉ ET PROGRAMME D'ORDINATEUR POUR LA DÉTECTION DE DONNÉES D'IMAGES OPTIQUES ET POUR LA DÉTERMINATION D'UNE POSITION D'UNE LIMITE LATÉRALE D'UN DISPOSITIF DE POSITIONNEMENT DE PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2016   DE 102016015121**

(43) Veröffentlichungstag der Anmeldung:
**30.10.2019   Patentblatt 2019/44**

(73) Patentinhaber: **Drägerwerk AG & Co. KGaA
23558 Lübeck (DE)**

(72) Erfinder:
• **FRANZ, Frank
  23617 Stockelsdorf (DE)**
• **SCHLICHTING, Stefan
  23562 Lübeck (DE)**
• **DIESEL, Jasper
  23564 Lübeck (DE)**

(56) Entgegenhaltungen:
WO-A1-2015/055312     US-A1- 2012 075 464
US-A1- 2012 223 821     US-A1- 2016 034 773

• LIU LIANG ET AL: "Bed angle detection in hospital room using Microsoft Kinect V2", 2016 IEEE 13TH INTERNATIONAL CONFERENCE ON WEARABLE AND IMPLANTABLE BODY SENSOR NETWORKS (BSN), IEEE, 14. Juni 2016 (2016-06-14), Seiten 277-280, XP032925857, DOI: 10.1109/BSN.2016.7516273 [gefunden am 2016-07-18]
• PANACHIT KITTIPANYA-NGAM ET AL: "Bed detection for monitoring system in hospital wards", ENGINEERING IN MEDICINE AND BIOLOGY SOCIETY (EMBC), 2013 35TH ANNUAL INTERNATIONAL CONFERENCE OF THE IEEE, IEEE, 28. August 2012 (2012-08-28), Seiten 5887-5890, XP032464274, ISSN: 1557-170X, DOI: 10.1109/EMBC.2012.6347333

**Beschreibung**

Ausführungsbeispiele beziehen sich auf eine Vorrichtung, ein Verfahren und ein Computerprogramm zur Bestimmung einer Lage einer Seitenbegrenzung einer Patientenlagerungsvorrichtung, insbesondere aber nicht ausschließlich, auf ein Konzept zur automatisierten Bestimmung der Lage einer Seitenbegrenzung eines Krankenbettes basierend auf optischen Bilddaten.

In der konventionellen Technik sind verschiedene Konzepte bekannt, die eine Lage, Haltung bzw. Liegehaltung oder Position/Pose eines Patienten auf einem Krankenbett abschätzen, beispielsweise motiviert dadurch, dass ungünstige Köperhaltungen existieren, die einen Genesungs- oder Heilprozess negativ beeinflussen oder ein Gesundheitsrisiko darstellen können. Darunter kann auch ein Verharren in einer Position oder Haltung über eine gewisse Zeitdauer fallen. Für an ein Krankenbett gebundene Patienten hängt deren Haltung oder Pose von einer Einstellung oder Konfiguration des benutzten Krankenbettes ab. Solche Patienten befinden sich oftmals in Situationen, beispielsweise in dafür vorgesehenen Unterbringungen, Stationen oder Krankenzimmern, in welchen dann entsprechende Überwachung, Dokumentation und Warnmechanismen vorgesehen sind, um kritische oder Fehlhaltungen zu vermeiden. Einrichtungen für betreutes Wohnen, Pflegeeinrichtungen, Heimpflegeplätze, Altenheime, Krankenhäuser und Intensivstationen sind einige Beispiele.

Eine wichtige Rolle dabei spielen auch etwaige Seitenbegrenzungen, wie etwa Bettgitter, Schutzgitter, Begrenzungen, usw., die an Lagerungsvorrichtungen für Patienten angebracht sind, und die beispielsweise zur Sicherung der Patienten gegen Heraus- oder Herunterfallen eingesetzt werden.

Im Bereich der Pflege gibt es einstellbare oder konfigurierbare Kranken- oder Pflegebetten, die Patienten zu Hause oder auch in entsprechenden Einrichtungen wie Krankenhäusern zur Verfügung stehen. Die verfügbaren Krankenbetten sind zumeist nicht in der Lage Information über eine derzeitige Konfiguration zur Verfügung zu stellen, bzw. verwenden dazu herstellerspezifische oder eigene Protokolle. Das Verhindern von Stürzen und Einklemmungen ist in der medizinischen Betreuung von hohem Interesse. Personen, die für diese Art von Unfällen besonders anfällig sind, leiden häufig unter einer Krankheit wie Alzheimer, sind körperlich beeinträchtigt oder stehen unter dem Einfluss starker Medikamente. Insbesondere auf der Intensivstation kommt es zu unkoordiniertem, unbewussten bis bewusst autoaggressivem Handeln der Patienten im Delirium ("Durchgangssyndrom"), welches immer wieder zu Stürzen des ohnehin schwer Erkrankten aus der Patientenlagerungsvorrichtung (PLV) führt. Dies kann drastische Folgen von schweren Verletzungen bis zum Tod haben.

Neben Intensivstationen befinden sich die Patienten in Krankenhäusern, Seniorenheimen, psychiatrischen Anstalten oder anderen Pflegeeinrichtungen. Das Pflegepersonal kann verschiedene Gegenmaßnahmen ergreifen, die das Risiko eines Unfalls verringern sollen. Dazu gehören besonders die ständige Überwachung und korrekte Einschätzung des Patienten, eine sichere Umgebung und verschiedene Hilfsmittel wie Bettgitter an PLVs und deren Alternativen.

Das Dokument US 2012/0075464 A1 offenbart ein System zur Überwachung eines Patienten sowie des Zustands des Krankenbetts mittels Videokameras. Das System kann feststellen, ob die Seitenbegrenzungen des Krankenbetts angehoben oder abgesenkt sind.

Weitere Hintergrundinformation kann in den folgenden Schriften gefunden werden:

- Besl, P. J. (1992), "Method for registration of 3-D shapes", In Robotics-DL tentative (pp. 586-606),
- Fischler, M. A. (1981), "Random sample consensus: a paradigm for model fitting with applications to image analysis and automated cartography", Communications of the ACM, pp. 381-395,
- Hartman, F. (2011), "Robotersteuerung durch Gesten", Masterarbeit Universität zu Lübeck,
- Kong, T., & Rosenfeld, A. (1996), "Topological Algorithms for Digital Image Processing", Elsevier Science, Inc.,

- Shapiro, L., & Stockman, G. (2001), "Computer Vision", Prentice-Hall,
- Bron, C., & Kerbosch, J. (1973), "Algorithm 457: finding all cliques of an undirected graph, Communications of the ACM" , S. 575-577,
- Canny, J. (1986), "A Computational Approach to Edge Detection", IEEE Transactions on Pattern Analysis and Machine Intelligence,
- Capezuti, E., Wagner, L., & Brush, B. (2007), "Consequences of an intervention to reduce restrictive side rail use in nursing homes", Journal of the American Geriatrics Society, S. 334-341,
- Dalal, N., & Triggs, B. (2005), "Histograms of Oriented Gradients for Human Detection", IEEE Computer Society Conference on Computer Vision and Pattern Recognition,
- Duda, R., & Hart, P. E. (1972), "Use ofthe Hough Transformation to Detect Lines and Curves in Pictures", Comm. ACM, S. 11-15,
- Hartman, F. (2011), "Robotersteuerung durch Gesten", Universität Lübeck,
- Jolliffe, I. T. (2002), "Principal Component Analysis", Springer,
- Soille, P. (1999), "Morphological Image Analysis: Principles and Applications", Springer-Verlag,
- Talerico, K. A., & Capezuti, E. (2001), "Myths and Facts About Side Rails: Despite ongoing debates about safety and efficacy, side rails are still a standard component of care in many hospitals, so how do you determine their safe use?", AJN The American Journal of Nursing, S. 43-48, und

- Viola, P., & Jones, M. (2001), "Rapid object detection using a boosted cascade of simple features", CONFERENCE ON COMPUTER VISION AND PATTERN RECOGNITION 2001.

Es besteht daher ein Bedarf ein verbessertes Konzept für die Erkennung einer Bettkonfiguration zu schaffen. Diesem Bedarf werden Ausführungsbeispiele einer Vorrichtung, eines Verfahrens und eines Computerprogramms gemäß den anhängigen unabhängigen Ansprüchen gerecht.

Manche Ausführungsbeispiele können beispielsweise automatisch die Einstellung von Bettgittern (und damit auch deren Einsatz) erkennen, um beispielsweise bei der Dokumentation zu assistieren oder weitere Überwachungssysteme zu aktivieren, die beim Einsatz von Bettgittern angezeigt sind. Dies wird dadurch gelöst, dass optische Bilddaten einer Patientenlagerungsvorrichtung erfasst werden, dass ferner eine Lage von zumindest einem Teilsegment der Patientenlagerungsvorrichtung und basierend darauf eine Lage einer Seitenbegrenzung der Patientenlagerungsvorrichtung bestimmt wird. Aus der Lage der Seitenbegrenzung lassen sich Rückschlüsse auf die Situation des Patienten ziehen. Beispielsweise kann auf Sicherheitsrisiken geschlossen werden, wenn eine Seitenbegrenzung nicht hoch genug eingestellt ist.

Die vorgeschlagene Lösung ist insbesondere deshalb vorteilhaft, da von der Lage des zumindest einen Teilsegmentes und der Seitenbegrenzung auf ein Risiko für den Patienten geschlossen werden kann. Mit anderen Worten: Ausführungsbeispiele der vorliegenden Erfindung basieren auf dem Gedanken, optisch erfasste Bilddaten einer Patientenlagerungsvorrichtung auszuwerten bzw. zu verarbeiten und daraus auf die Lage zumindest eines Teilsegmentes und einer Seitenbegrenzung zu schlie-ßen. Ausführungsbeispiele schaffen eine Vorrichtung zur Erfassung von optischen Bilddaten einer Patientenlagerungsvorrichtung und zur Bestimmung einer Lage von zumindest einer Seitenbegrenzung der Patientenlagerungsvorrichtung basierend auf den Bilddaten. Die Vorrichtung ist ausgebildet, um zunächst eine Lage von zumindest einem Teilsegment der Patientenlagerungsvorrichtung basierend auf den Bilddaten zu bestimmen. Die Vorrichtung ist ferner ausgebildet, um basierend auf der Lage des zumindest einen Teilsegmentes die Lage der zumindest einen Seitenbegrenzung der Patientenlagerungsvorrichtung zu bestimmen. Ausführungsbeispiele können durch die Bestimmung der Lage der Seitenbegrenzung aus den Bilddaten ein verbessertes Konzept für die Beobachtung, die Sicherheitsüberwachung oder die Dokumentation der Konfigurationen von Patientenlagerungsvorrichtungen schaffen. In manchen Ausführungsbeispielen kann die Vorrichtung ferner eine Schnittstelle zur Ausgabe einer Information über die Lage der zumindest einen Seitenbegrenzung aufweisen. Beispielsweise kann so die Information über die Lage der Seitenbegrenzung verfügbar gemacht werden, z.B. zur Anzeige für Pflegepersonal, zur weiteren Datenverarbeitung oder zur automatisierten Überwachung.

In einigen Ausführungsbeispielen kann die Vorrichtung darüber hinaus eine Erfassungseinrichtung zur Erfassung der optischen Bilddaten der Patientenlagerungsvorrichtung umfassen. Die Erfassungseinrichtung kann einen oder mehrere Sensoren aufweisen, der/die zur Erfassung einer drei-dimensionalen Punktewolke als Bilddaten ausgebildet ist/sind. Insofern können die Bilddaten unabhängig von den Patientenlagerungsvorrichtungen erfasst werden, sodass beliebige Patientenlagerungsvorrichtungen eingesetzt werden können. Darüber hinaus kann auf an einer Patientenlagerungsvorrichtung angebrachte Sensorik verzichtet werden. Einschränkungen für Patienten oder Personal durch zusätzliche Komponenten oder Verkabelungen an eine Patientenlagerungsvorrichtung können so vermieden werden.

Die Vorrichtung umfasst ferner eine Bestimmungseinrichtung, die zur Bestimmung der Lage des zumindest einen Teilsegmentes der Patientenlagerungsvorrichtung basierend auf den Bilddaten ausgebildet ist. Die Bestimmungseinrichtung kann ferner ausgebildet sein, um basierend auf der Lage des zumindest einen Teilsegmentes der Patientenlagerungsvorrichtung eine Größe und eine Position einer Liegefläche der Patientenlagerungsvorrichtung zu bestimmen. Ausführungsbeispiele können so Information über die Liegefläche, z.B. deren Ausrichtung oder Konfiguration mit in die weiteren Betrachtung einbeziehen.

Beispielsweise kann die Bestimmungseinrichtung ausgebildet sein, um ferner zwei Längsseiten der Patientenlagerungsvorrichtung zu bestimmen, und basierend auf den Längsseiten aus den Bilddaten Bildpunkte zumindest teilweise auszuschließen, die nicht zu der zumindest einen Seitenbegrenzung der Patientenlagerungsvorrichtung gehören. Ausführungsbeispiele können so eine effektive Datenverarbeitung ermöglichen. Die Bestimmungseinrichtung kann darüber hinaus ausgebildet sein, um die Bilddaten ferner auf Bildpunkte einer Längsseite der Patientenlagerungsvorrichtung einzuschränken. Eine Detektion der Seitenbegrenzungen kann so für beispielsweise zwei Längsseiten getrennt erfolgen und so eine Detektion erleichtern. Die Bestimmungseinrichtung kann ferner ausgebildet sein, um die Bilddaten auf eine Seitenebene der Patientenlagerungsvorrichtung zu projizieren und um ein Projektionsbild zu erhalten. Die Bestimmungseinrichtung kann dann ausgebildet sein, um in dem Projektionsbild mittels einer Objekt-Detektion Seitenbegrenzungskandidaten zu bestimmen. Ausführungsbeispiele können so einen effektiven Algorithmus zur Auswertung der Bilddaten bereitstellen. In weiteren Ausführungsbeispielen kann die Bestimmungseinrichtung ausgebildet sein, um die Seitenbegrenzungskandidaten zu bewerten und um basierend auf den bewerteten Seitenbegrenzungskandidaten die Lage der zumindest einen Seitenbegrenzung zu bestimmen. Eine Zuverlässigkeit der Detektion der Seitenbegrenzung kann durch die Bewertung gemessen und in eine Betrachtung mit einbezogen werden.

Die Bestimmungseinrichtung ist ausgebildet, um basierend auf der Lage der zumindest einen Seitenbegrenzung Sicherheitsinformation über eine Konfiguration der Patientenlagerungsvorrichtung zu bestimmen. Die Sicherheitsinformation kann dann zur Beurteilung einer Situation für den Patienten herangezogen werden. Beispielsweise kann die Bestimmungseinrichtung ausgebildet sein, um die Sicherheitsinformation über eine Speichereinrichtung zu dokumentieren und/oder um die Sicherheitsinformation über eine Darstellungseinrichtung auszugeben. Ausführungsbeispiele können die Sicherheitsinformation so verfügbar machen. Zumindest in manchen Ausführungsbeispielen kann die Bestimmungseinrichtung auch ausgebildet sein, um basierend auf der Sicherheitsinformation eine Alarmierungsinformation auszugeben, wenn die Konfiguration der Patientenlagerungsvorrichtung ein Sicherheitsniveau unterschreitet. Pflegepersonal kann dann alarmiert werden. Z.B. kann das Sicherheitsniveau der Patientenlagerungsvorrichtung auf einer relativen Lage des zumindest einen Teilsegmentes zu der zumindest einen Seitenbegrenzung basieren. Die relative Lage des zumindest einen Teilsegmentes bzw. einer parallel dazu angeordneten Liegefläche relativ zu der Seitenbegrenzung kann beispielsweise ein Indikator für ein Risiko eines seitlichen Herausrutschens, -rollens oder -fallens sein. Das Sicherheitsniveau kann ein Maß dafür darstellen, wie hoch ein Risiko eines Herausfallens eines Patienten aus der Patientenlagerungsvorrichtung bei der bestimmten Konfiguration der Patientenlagerungsvorrichtung ist. Dadurch kann wiederum eine Dokumentation oder ein Frühwarnung für das Pflegepersonal aufgebaut werden. Das Sicherheitsniveau stellt ein Maß einer mittleren Höhe der Seitenbegrenzung über einer Liegefläche über dem zumindest einen Teilsegment dar.

In weiteren Ausführungsbeispielen kann die Bestimmungseinrichtung auch ausgebildet sein, um die Lage von zumindest zwei Teilsegmenten, z.B. auch vier, der Patientenlagerungsvorrichtung zu bestimmen. Ausführungsbeispiele können so Sicherheitsinformation für verschiedene Konfigurationen auch bei komplizierteren Patientenlagerungsvorrichtungen verfügbar machen.

Ausführungsbeispiele schaffen darüber hinaus ein Verfahren zur Erfassung von optischen Bilddaten einer Patientenlagerungsvorrichtung und zur Bestimmung einer Lage von zumindest einer Seitenbegrenzung der Patientenlagerungsvorrichtung basierend auf den Bilddaten. Das Verfahren umfasst ein Bestimmen einer Lage von zumindest einem Teilsegment der Patientenlagerungsvorrichtung basierend auf den Bilddaten. Das Verfahren umfasst ferner ein Bestimmen der Lage der zumindest einen Seitenbegrenzung der Patientenlagerungsvorrichtung basierend auf der Lage des zumindest einen Teilsegmentes.

Ein weiteres Ausführungsbeispiel ist ein Programm oder Computerprogramm mit einem Programmcode zur Durchführung eines hierin beschriebenen Verfahrens, wenn der Programmcode auf einem Computer, einem Prozessor oder einer programmierbaren Hardwarekomponente ausgeführt wird.

Weitere vorteilhafte Ausgestaltungen werden nachfolgend anhand der in den Zeichnungen dargestellten Ausführungsbeispiele, auf welche Ausführungsbeispiele generell jedoch nicht insgesamt beschränkt sind, näher beschrieben. Es zeigen:

Fig. 1 ein Ausführungsbeispiel einer Vorrichtung zur Erfassung von optischen Bilddaten einer Patientenlagerungsvorrichtung und zur Bestimmung einer Lage von zumindest einer Seitenbegrenzung;

Fig. 2 ein Krankenbett als Patientenlagerungsvorrichtung mit vier Segmenten in einem Ausführungsbeispiel;

Fig. 3 ein Krankenbett als Patientenlagerungsvorrichtung mit vier Seitenbegrenzungen oder Bettgittern;

Fig. 4 ein Übersichtsdiagramm zur Bestimmung von dreidimensionalen Bilddaten in einigen Ausführungsbeispielen;

Fig. 5 ein Ausführungsbeispiel in einem Krankenzimmer;

Fig. 6 ein Ablaufdiagramm zur Bestimmung der Lage der Seitenbegrenzung in einem Ausführungsbeispiel;

Fig. 7 erfasste Bilddaten mit einem Begrenzungsrahmen in einem Ausführungsbeispiel;

Fig. 8 erfasste Bilddaten mit einem Begrenzungsrahmen und bestimmten Teilsegmenten in einem Ausführungsbeispiel;

Fig. 9 ein Ablaufdiagramm zur Bestimmung der Seitenbegrenzung in einem Ausführungsbeispiel;

Fig. 10 eine Illustration zweier Histogramme in einem Ausführungsbeispiel;

Fig. 11 eine Illustration zweier Histogramme in einem Ausführungsbeispiel zusammen mit gefundenen Geraden (links) und mittleren Geraden (rechts);

Fig. 12 basierend auf Seitenebenen gewonnene Begrenzungsrahmen in einem Ausführungsbeispiel;

Fig. 13 eine Illustration eines Projektionsbildes in einem Ausführungsbeispiel unter Kenntnis einer Orientierung einer Liegefläche einer Patientenlagerungsvorrichtung;

Fig. 14 von einem Objekt-Detektor identifizierte Seitenbegrenzungen in einem Ausführungsbeispiel;

Fig. 15 mögliche Konfigurationen eines Krankenbettes mit Bettgittern in einem Ausführungsbeispiel;

Fig. 16 potentielle Bewertungen in einem Ausführungsbeispiel;

Fig. 17 verschiedene Ausführungsbeispiele von Verfahren zur Bestimmung einer Sicherheitsinformation; und

Fig. 18 ein Blockschaltbild eines Ablaufdiagramms eines Ausführungsbeispiels eines Verfahrens zur Bestimmung einer Seitenebene.

Verschiedene Ausführungsbeispiele werden nun ausführlicher unter Bezugnahme auf die beiliegenden Zeichnungen beschrieben, in denen einige Ausführungsbeispiele dargestellt sind.

Bei der nachfolgenden Beschreibung der beigefügten Figuren, die lediglich einige exemplarische Ausführungsbeispiele zeigen, können gleiche Bezugszeichen gleiche oder vergleichbare Komponenten bezeichnen. Ferner können zusammenfassende Bezugszeichen für Komponenten und Objekte verwendet werden, die mehrfach in einem Ausführungsbeispiel oder in einer Zeichnung auftreten, jedoch hinsichtlich eines oder mehrerer Merkmale gemeinsam beschrieben werden. Komponenten oder Objekte, die mit gleichen oder zusammenfassenden Bezugszeichen beschrieben werden, können hinsichtlich einzelner, mehrerer oder aller Merkmale, beispielsweise ihrer Dimensionierungen, gleich, jedoch gegebenenfalls auch unterschiedlich ausgeführt sein, sofern sich aus der Beschreibung nicht etwas anderes explizit oder implizit ergibt. Optionale Komponenten sind in den Figuren mit gestrichelten Linien oder Pfeilen dargestellt.

Obwohl Ausführungsbeispiele auf verschiedene Weise modifiziert und abgeändert werden können, sind Ausführungsbeispiele in den Figuren als Beispiele dargestellt und werden hierin ausführlich beschrieben. Es sei jedoch klargestellt, dass nicht beabsichtigt ist, Ausführungsbeispiele auf die jeweils offenbarten Formen zu beschränken, sondern dass Ausführungsbeispiele vielmehr sämtliche funktionale und/oder strukturelle Modifikationen, Äquivalente und Alternativen, die im Bereich der Erfindung liegen, abdecken sollen. Gleiche Bezugszeichen bezeichnen in der gesamten Figurenbeschreibung gleiche oder ähnliche Elemente.

Man beachte, dass ein Element, das als mit einem anderen Element "verbunden" oder "verkoppelt" bezeichnet wird, mit dem anderen Element direkt verbunden oder verkoppelt sein kann oder dass dazwischenliegende Elemente vorhanden sein können. Wenn ein Element dagegen als "direkt verbunden" oder "direkt verkoppelt" mit einem anderen Element bezeichnet wird, sind keine dazwischenliegenden Elemente vorhanden. Andere Begriffe, die verwendet werden, um die Beziehung zwischen Elementen zu beschreiben, sollten auf ähnliche Weise interpretiert werden (z.B., "zwischen" gegenüber "direkt dazwischen", "angrenzend" gegenüber "direkt angrenzend" usw.).

Die Terminologie, die hierin verwendet wird, dient nur der Beschreibung bestimmter Ausführungsbeispiele und soll die Ausführungsbeispiele nicht beschränken. Wie hierin verwendet, sollen die Singularformen "einer," "eine", "eines" und "der, die, das" auch die Pluralformen beinhalten, solange der Kontext nicht eindeutig etwas anderes angibt. Ferner sei klargestellt, dass die Ausdrücke wie z.B. "beinhaltet", "beinhaltend", "aufweist", "umfasst", "umfassend" und/oder "aufweisend", wie hierin verwendet, das Vorhandensein von genannten Merkmalen, ganzen Zahlen, Schritten, Arbeitsabläufen, Elementen und/oder Komponenten angeben, aber das Vorhandensein oder die Hinzufügung von einem bzw. einer oder mehreren Merkmalen, ganzen Zahlen, Schritten, Arbeitsabläufen, Elementen, Komponenten und/oder Gruppen davon nicht ausschließen.

Solange nichts anderes definiert ist, haben sämtliche hierin verwendeten Begriffe (einschließlich von technischen und wissenschaftlichen Begriffen) die gleiche Bedeutung, die ihnen ein Durchschnittsfachmann auf dem Gebiet, zu dem die Ausführungsbeispiele gehören, beimisst. Ferner sei klargestellt, dass Ausdrücke, z.B. diejenigen, die in allgemein verwendeten Wörterbüchern definiert sind, so zu interpretieren sind, als hätten sie die Bedeutung, die mit ihrer Bedeutung im Kontext der einschlägigen Technik konsistent ist, und nicht in einem idealisierten oder übermäßig formalen Sinn zu interpretieren sind, solange dies hierin nicht ausdrücklich definiert ist.

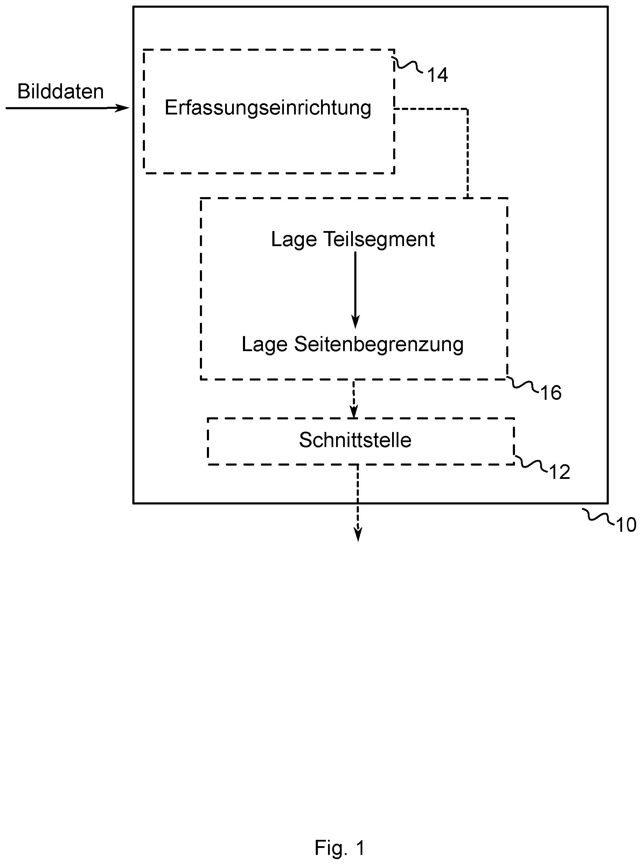

Fig. 1 zeigt ein Ausführungsbeispiel einer Vorrichtung 10 zur Erfassung von optischen Bilddaten einer Patientenlagerungsvorrichtung und zur Bestimmung einer Lage von zumindest einer Seitenbegrenzung der Patientenlagerungsvorrichtung basierend auf den Bilddaten. Die Vorrichtung 10 ist ausgebildet, um zunächst eine Lage von zumindest einem Teilsegment der Patientenlagerungsvorrichtung basierend auf den Bilddaten zu bestimmen, und um basierend auf der Lage des zumindest einen Teilsegmentes die Lage der zumindest einen Seitenbegrenzung der Patientenlagerungsvorrichtung zu bestimmen. Optionale Komponenten sind in den Figuren mit gestrichelten Linien dargestellt.

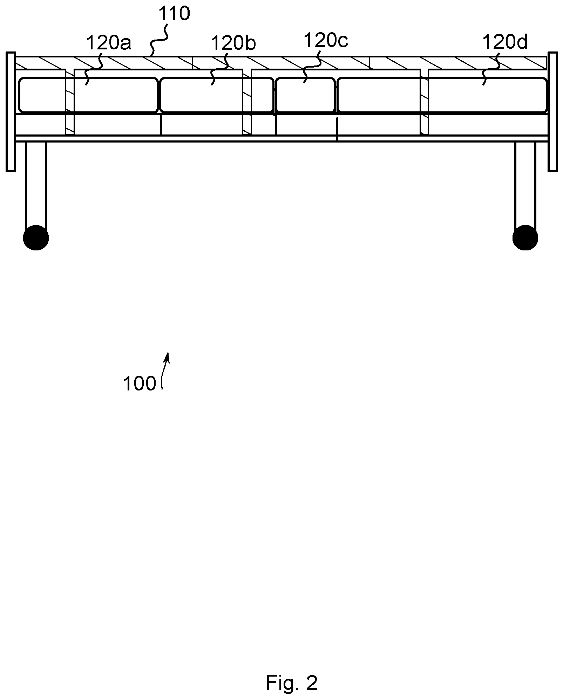

In Ausführungsbeispielen kann die Patientenlagerungsvorrichtung 100 ein oder mehrere Segmente 120 aufweisen. Fig. 2 zeigt ein Krankenbett als Beispiel einer Patientenlagerungsvorrichtung 100 mit vier Segmenten 120a, 120b, 120c und 120d. Hier und im Folgenden sei unter einer Patientenlagerungsvorrichtung 100 beispielsweise ein verstellbares Krankenbett, ein Operationstisch, eine Liege, eine Bare, ein Schleusen-Tisch, ein Krankenstuhl, ein Rollstuhl, usw. verstanden, also eine Vorrichtung, die sich zur Lagerung, zum Betten, zum Unterstützen, evtl. zum Transportieren usw. von Personen, Kranken oder Pflegebedürftigen eignet. Im Folgenden werden einige Ausführungsbeispiele am Beispiel eines Krankenbettes betrachtet. Dieses Krankenbett ist als Stellvertreter für beliebige Patientenlagerungsvorrichtungen gedacht und zu verstehen.

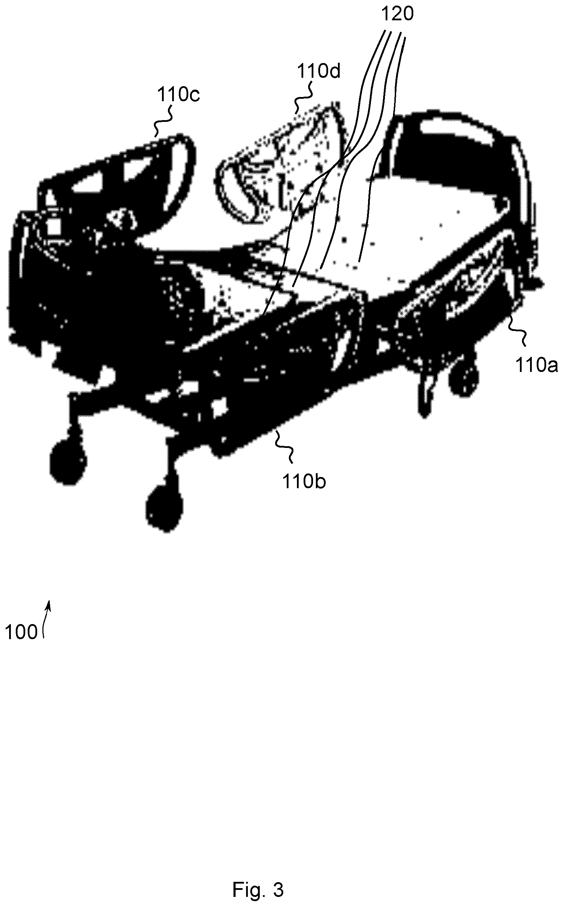

Fig. 3 zeigt ein Krankenbett als Patientenlagerungsvorrichtung 100 mit vier Seitenbegrenzungen oder Bettgittern 110a, 110 b, 110c, 110 d und mehreren Teilsegmenten 120. Wie die Fign. 2 und 3 zeigen, können die Segmente 120 dabei für unterschiedliche Funktionen vorgesehen sein, beispielsweise Fuß-, Bein-, Rumpf-, Rücken-, Kopfunterstützung, usw., sowie in Bereiche unterteilt sein, z.B. Sitz- und Liegebereich. Die Teilsegmente 120, 120a, 120b, 120c, 120d in der Fig. 2 sind zumindest teilweise Segmenten eines Liege- und/oder Sitzbereiches der Patientenlagerungsvorrichtung 100 zugeordnet. In weiteren Ausführungsbeispielen kann anstatt des Krankenbettes, auch eine beliebige andere Patientenlagerungsvorrichtung 100 mit entsprechenden Segmenten 120 betrachtet werden. Die Teilsegmente 120a, 120b, 120c, 120d können konfigurierbar oder verstellbar sein und daher verschiedene Lagen zueinander oder auch relativ zu einem Bettgestell einnehmen, vgl. Fign. 2 und 3.

In dem gezeigten Ausführungsbeispiel ist die Matratze des Bettes in die vier Segmente 120a, 120b, 120c, 120d unterteilt, die konfigurierbar sind. In der Fig. 2 sind die Seitenbegrenzungen 110 als hochgeklappte Bettgitter dargestellt, die zumindest bei flacher Einstellung der Teilsegmente 120a, 120b, 120c und 120d eine gewisse Sicherung eines Patienten gegen Herausrollen bieten. Hier und im Folgenden seien unter der Lage eines Segmentes 120, 120a, 120b, 120c, 120d oder einer Seitenbegrenzung dessen/deren Orientierung, Richtung, relative Ausrichtung und Position zu zumindest einem anderen Teilsegment, z.B. Schnittwinkel der Längs- oder Querachsen, relative Ausrichtung und Position gegenüber einem Referenzobjekt, z.B. Untergrund, Achse der Patientenlagerungsvorrichtung, Information, die dem Pflegepersonal Aufschluss darüber gibt, ob eine Einstellung der Patientenlagerungsvorrichtung oder einer Seitenbegrenzung bei gegebenem Zustand eines darauf befindlichen Patienten geändert werden soll, usw. verstanden. Die Bestimmung einer Information über die Lage wird also mit dem Ziel durchgeführt, eine Aussage über die Einstellung oder Konfiguration der Patientenlagerungsvorrichtung 100 zu machen, um dann nachfolgend diese beispielsweise dokumentieren zu können und/oder auch einschätzen zu können, ob eine Änderung der Einstellung erfolgen sollte. Insofern kann die Lage des zumindest einen Teilsegmentes 120 und der zumindest einen Seitenbegrenzung 110 sich auf jegliche ein-, zwei- oder dreidimensionale Information beziehen, die einen Rückschluss auf die Einstellung oder Konfiguration des zumindest einen Teilesegmentes 120 oder der zumindest einen Seitenbegrenzung 110 zulässt, z.B. in Form von Winkeln, Geraden, Ebenen, usw.

Fig. 3 zeigt verschiedene Lagen von Seitenbegrenzungen 110a, 110b, 110c und 110d in Ausführungsbeispielen. Die Konfigurationen sind Beispielkonfigurationen von verstellbaren Seitenbegrenzungen, vier in diesem Ausführungsbeispiel.

Die Fig. 1 zeigt darüber hinaus, dass die Vorrichtung 10 in manchen Ausführungsbeispielen ferner eine Schnittstelle 12 zur Ausgabe einer Information über die Lage der zumindest einen Seitenbegrenzung 110 aufweisen kann. Die Schnittstelle 12 kann mit einer weiter unten erläuterten Bestimmungseinrichtung 16 gekoppelt sein. Über die Schnittstelle 12 kann beispielsweise Information über die Konfiguration/Lage des zumindest einen Teilsegmentes 120 und/oder der Seitenbegrenzung 110 (z.B. Winkel, Schnittwinkel, davon abgeleitete Information usw.) und/oder Information über die Zuverlässigkeit dieser Information an andere Komponenten kommuniziert werden, z.B. zur nachfolgenden weiteren Verarbeitung der Bilddaten, beispielsweise an ein Display oder an einen Monitor, eine Darstellungseinrichtung, eine Speichereinrichtung, eine Alarmierungseinrichtung oder ein Dokumentationssystem.

Die Schnittstelle 12 kann beispielsweise einem oder mehreren Eingängen und/oder einem oder mehreren Ausgängen zum Empfangen und/oder Übertragen von Informationen entsprechen, etwa in digitalen Bitwerten, analogen Signalen, Magnetfeldern, basierend auf einem Code, innerhalb eines Moduls, zwischen Modulen, oder zwischen Modulen verschiedener Entitäten. Die Schnittstelle 12 kann aber auch einer Eingabeschnittstelle 12 entsprechen, wie etwa einem Bedienfeld, einem Schalter oder Drehschalter, einem Knopf, einem berührungsempfindlichen Bildschirm (auch englisch "Touchscreen") usw. Die Schnittstelle 12 erlaubt somit das Aufnehmen, ggf. auch Empfang oder Eingabe, einer Information, beispielsweise darüber, ob eine Bestimmung der Teilsegmentlagen durchgeführt werden soll.

PLVs 100 werden in vielen medizinischen Bereichen, wie in Kliniken (z.B. in Notaufnahme, Aufwachraum, Patientenzimmer, Intensivstation), im Rettungsdienst oder in Seniorenheimen und in der häuslichen Pflege, eingesetzt. Sie zeichnen sich allgemein dadurch aus, dass sie dem Patienten eine Liegefläche bzw. Sitzfläche bieten. An PLVs 100 werden oftmals besondere Anforderungen gestellt, die speziell auf die Bedürfnisse von Patienten zugeschnitten sind. So ist die Liegefläche häufig konfigurierbar, da sie aus verschiedenen gegeneinander verstellbaren (PLV-)Segmenten 120, 120a, 120b, 120c, 120d besteht. Die Bestimmungseinrichtung 16 kann ausgebildet sein, um die Lage von zumindest einem Teilsegment 120 der Patientenlagerungsvorrichtung 100 zu bestimmen. Im Folgenden werden Ausführungsbeispiele betrachtet, in denen es vier Teilsegmente 120a-d gibt, in anderen Ausführungsbeispielen können auch mehr oder weniger Teilsegmente 120 vorkommen.

Ein derartiges Beispiel ist in Fig. 2 zu sehen. Hier ist das Kopfteil der Liegefläche aufrichtbar und die Segmente 120c, 120d der Beinpartie so einstellbar, dass die Beine des Patienten angewinkelt werden können. Somit lässt sich die Lage eines Patienten maßgeblich beeinflussen, was Komfort erhöhen, Risiken minimieren und Therapieresultate verbessern kann. Des Weiteren sind PLVs 100 häufig insgesamt bis zu einem gewissen Grad höhenverstellbar und neigbar.

Viele PLVs 100 sind zusätzlich mit Seitenbegrenzungen 110, meist auf beiden Seiten, ausgestattet. Sie sollen dem Schutz des Patienten dienen und ein Herausrollen/fallen aus der PLV verhindern. Die wohl häufigste Form der Seitenbegrenzung ist das Bettgitter (oder auch "Bettseitenstützen" oder "Bettseitenteile"), wie es auch in den Fign. 2 und 3 gezeigt ist. Bettgitter werden seit etwa 70 Jahren zur Prävention von Stürzen verwendet. Mögliche Ausführungen unterscheiden sich oft darin, ob sie auf beiden Bettseiten durchgängig vorhanden sind, oder ob je Bettseite zwei voneinander getrennte Bettgitter platziert sind. Ein Beispiel für erstere Variante ist in Fig. 2 zu sehen, ein Beispiel für letztere in Fig. 3. Die Bettgitter sind meist entweder direkt seitlich am Bettgestell befestigt oder an einem der PLV-Segmente 120. Sie sind hoch- und tiefverstellbar, potentiell auch stufenlos auf eine Einstellung dazwischen.

Im Ausführungsbeispiel der Fig. 3 sind die Bettgitter pro Seite zweiteilig, und an den PLV-Segmenten 120 befestigt. Auf der linken Seite der PLV 100 sind sie hochgestellt, auf der rechten tiefgestellt.

Stürze passieren aber auch trotz des Einsatzes von Bettgittern. Sie können sogar in ungünstigen Fällen die Schwere eines möglichen Sturzes erhöhen, da der Patient beim Versuch sie zu überwinden eine höhere Fallhöhe hat. Zusätzlich besteht das Risiko, dass sich ein Patient ungünstig im Bettgitter einklemmt und in Folge dessen Verletzungen erleidet oder sogar stirbt. Längeres Aufliegen von Körperteilen, insbesondere den Extremitäten, kann in der Folge zu Druckgeschwüren führen, welche selbst wiederum ein hohes Gesundheitsrisiko darstellen. Dass Bettgitter also durchaus selbst ein (erhöhtes) Risiko darstellen können, wird auch in der Studie (Capezuti, Wagner, & Brush, 2007) thematisiert.

Bettgitter können einem Patienten ebenfalls helfen sich im Bett umzudrehen oder anders zu positionieren, da der Patient sich an ihnen festhalten und entlang ziehen kann. Außerdem bieten sie die Möglichkeit, Gegenstände zu befestigen (z.B. Drainagen, Urinbeutel, Patientenfixierungsmittel, usw.). In jedem Fall muss das Pflegepersonal gut abwägen, ob Bettgitter angebracht sind oder nicht. Eine mögliche Leitlinie, insbesondere für die USA, zur Entscheidung findet sich z.B. in (Talerico & Capezuti, 2001). Zumindest in Deutschland gibt es auch juristische Hürden für den Einsatz von Bettgittern, denn das Hochstellen kann eine Freiheitsberaubung darstellen, weshalb ein Rechtfertigungsgrund von Nöten sein kann. Ist ein Patient zurechnungsfähig, kann er selbst den Grund liefern. Sonst darf ein Arzt in einer Notfallsituation den Einsatz von Bettgittern für maximal 24 Stunden anordnen. Auch in einer Pflegesituation (z.B. beim Verändern der Einstellung oder Position der PLV 100) ist es erlaubt Bettgitter kurzfristig hochzustellen. Andernfalls oder darüber hinaus müsste ggf. ein Richter konsultiert werden.

Das Hochstellen von Bettgittern bedarf jedes Mal einer Dokumentation. Diese sollte mindestens den Zeitpunkt, die Begründung und einen Hinweis auf die Genehmigung umfassen. Für weitere Details in Bezug auf PLVs 100 und auch hinsichtlich der weiteren hierin erläuterten Aspekte wird auf das Dokument DE 10 2015 013 031.5 verwiesen, dass sich mit der Bestimmung von Teilsegmentlagen einer PLV 100 basierend auf Bilddaten beschäftigt, jedoch nicht auf die mit einer Seitenbegrenzung 110 verbundene Problematik eingeht.

Wie die Fig. 1 weiter illustriert kann die Vorrichtung 10 in weiteren Ausführungsbeispielen eine Erfassungseinrichtung 14 zur Erfassung der optischen Bilddaten der Patientenlagerungsvorrichtung 100 umfassen. Die Erfassungseinrichtung 14 kann einen oder mehrere Sensoren aufweisen, der/die zur Erfassung einer dreidimensionalen Punktewolke als Bilddaten ausgebildet ist/sind, wie dies in der Fig. 1 optional dargestellt ist (optionale Komponenten sind in der Fig. 1 mit gestrichelten Linien dargestellt). Die Erfassungseinrichtung 14 kann mit einer Bestimmungseinrichtung 16 und/oder mit der Schnittstelle 12 gekoppelt sein. Die Erfassungseinrichtung 14 kann dabei ein oder mehreren beliebigen optischen Erfassungseinheiten, Erfassungsgeräten, Erfassungsmodulen, usw. entsprechen. Denkbar sind hier Kameras, Bildsensoren, Infrarotsensoren, Sensoren zur Erfassung ein-, zwei-, drei- oder mehr-dimensionaler Daten, Sensorelemente verschiedener Art usw. Die ein oder mehreren Sensoren können in weiteren Ausführungsbeispielen zumindest einen Sensor umfassen, der zumindest dreidimensionale Daten liefert. Die dreidimensionalen Daten erfassen demnach Information über Bildpunkte im Raum und können zusätzlich, quasi als weitere Dimensionen, weitere Information umfassen, beispielsweise Farbinformation (z.B. Rot, Grün, Blau (RGB) Farbraum), Infrarotintensität, Transparenzinformation (z.B. Alpha-Werte), usw.

Es gibt verschiedene Arten von Sensoren, die zwar kein zweidimensionales Bild einer Szene erzeugen, aber eine dreidimensionale Punktemenge erzeugen, etwa Bildpunkte mit Koordinaten oder verschiedener Tiefeninformation, die Information über Oberflächenpunkte eines Objektes umfassen.

Beispielsweise kann hier Information über einen Abstand der Bildpunkte zu dem Sensor oder Sensorsystem selbst vorhanden sein. Es gibt einige Sensoren, die nicht nur ein zweidimensionales Bild aufnehmen, sondern zusätzlich eine Tiefenkarte, die die Distanzen der einzelnen Pixel zum Sensorsystem selbst beinhaltet. Hieraus lässt sich dann auch eine dreidimensionale Punktewolke errechnen, die die aufgenommene Szene in 3D repräsentiert.

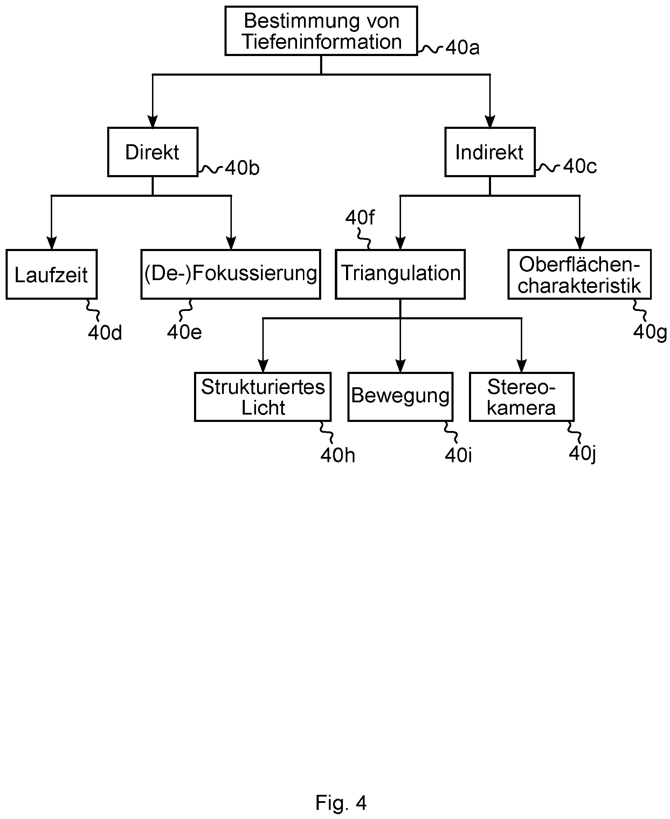

Eine Übersicht von den verschiedenen Methoden zur Bestimmung der Tiefeninformationen für die Tiefenkarte ist in Fig. 4 gezeigt. Man kann zwischen direkten und indirekten Methoden unterscheiden, wobei bei den ersteren die Distanz eines Punktes zum System direkt vom System selbst bestimmt wird und beim letzteren zusätzliche Verfahren benötigt werden. Zusätzliche Informationen über die einzelnen Möglichkeiten finden sich u.a. bei (Hartman, 2011). In jüngerer Vergangenheit sind diese Sensoren viel günstiger und besser geworden. Die dreidimensionale Information ermöglicht es Computern, aufgenommene Objekte genauer zu analysieren und interessante Informationen, wie Abstände zwischen Objekten, abzuleiten.

Die Fig. 4 zeigt ein Übersichtsdiagramm zur Bestimmung von dreidimensionalen Bilddaten in einigen Ausführungsbeispielen, wobei auch über die Fig. 4 hinausgehende Bestimmungsvarianten in Ausführungsbeispielen zum Einsatz kommen können. Es sei darauf hingewiesen, dass die dreidimensionalen Bilddaten auf die hier Bezug genommen wird, oftmals nur einem dreidimensionalen Teilbild entsprechen, da ein Sensor nur Bildpunkte aus einer bestimmten Perspektive bestimmt und somit ein unvollständiges dreidimensionales Bild entstehen kann. Wie im weiteren Verlauf noch erläutert wird, können auch mehrere solche Teilbilder zusammengefasst werden, um ein Bild mit verbesserter Qualität oder mehr Bildpunkten zu erhalten, das auch wiederum nur einem Teilbild entsprechen kann.

Die Fig. 4 zeigt zunächst in 40a die Bestimmung oder Berechnung von Tiefeninformation in Bilddaten. Dabei lassen sich direkte Verfahren im Zweig 40b und indirekte Verfahren im Zweig 40c differenzieren, wobei die ersteren den Abstand eines Punktes zum System über das System direkt bestimmen und die letzteren zusätzliche Einrichtungen zur Abstandsbestimmung benötigen. Direkte Verfahren sind beispielsweise Laufzeitmessungen (auch engl. "time of flight") 40d und (De-) Fokussierungsverfahren 40e. Indirekte Verfahren umfassen beispielsweise Triangulation 40f (beispielsweise über strukturiertes Licht 40h, Bewegung 40i oder Stereo-Kameras 40j) und Auswertung von Oberflächencharakteristika 40g.

Weitere Details zu den verschiedenen Möglichkeiten finden sich beispielsweise in Hartman F., 2011, s.o. Solche Sensoren sind in der Vergangenheit kostengünstiger geworden und wurden weiterentwickelt sowie leistungsstärker. Dreidimensionale Information kann einen Computer in die Lage versetzen, entsprechende Analysen der erfassten Objekte durchzuführen und entsprechende Daten bereitzustellen.

Ausführungsbeispiele können als Ergebnis einen Datensatz liefern, welcher zumindest eine Teillage von wenigstens einem Teilsegment 120, 120a, 120b, 120c, 120d einer Patientenlagerungsvorrichtung 100 als Teilebene im dreidimensionalen Raum indiziert. In manchen Ausführungsbeispielen können Teilebenen als zweidimensionale Teilebenen im dreidimensionalen Raum oder als eindimensionale Teilgeraden im zweidimensionalen Raum indiziert werden. Es kann bei der Teilgeradendarstellung als höheres Wissen angenommen werden, dass die Ebenen sich in dritter Dimension senkrecht zu den zweidimensionalen Koordinaten erstrecken. Für weitere Details wird auf die DE 10 2015 013 031.5 verwiesen.

Zumindest manche Ausführungsbeispiele stellen ein Verfahren, ein Computerprogramm und/oder eine Vorrichtung bereit, um automatisch, berührungslos und zumindest teilweise ohne Kommunikationsverbindung zwischen der PLV 100 und einem abnehmenden System ein Maß für die Sicherheit eines Patienten in einer Bettkonfiguration in Abhängigkeit detektierter Lagen von zumindest einer Seitenbegrenzung 110 und Teilsegmentlagen 120 bereitzustellen. Diese Aufgabe kann in einigen Ausführungsbeispielen sowohl bei Tag als auch bei Nacht gelöst werden, beispielsweise mittels Infrarotsensoren.

Die Vorrichtung 10, wie sie in der Fig. 1 dargestellt ist, kann in Ausführungsbeispielen 1...n (n = ganze positive Zahl) Sensoren umfassen oder nutzen, die im Wesentlichen unabhängig von den für den Menschen sichtbaren Beleuchtungsverhältnissen arbeiten. Die Sensoren können beispielsweise jeweils eine 3D-Punktewolke der Szene erstellen, die zu einer Punktewolke zusammengefasst werden können. Die Sensoren können so ausgerichtet sein, dass sich die zu überwachende Patientenlagerungsvorrichtung 100 zu einem großen Anteil im Sichtfeld befindet.

Wie die Fig. 1 weiter zeigt, kann die Vorrichtung 10 ferner eine Bestimmungseinrichtung 16 umfassen, die zur Bestimmung der Lage des zumindest einen Teilsegmentes 120 der Patientenlagerungsvorrichtung 100 basierend auf den Bilddaten ausgebildet ist. Die Bestimmungseinrichtung 16 kann ferner ausgebildet sein, um basierend auf der Lage des zumindest einen Teilsegmentes 120 der Patientenlagerungsvorrichtung 100 eine Größe und eine Position einer Liegefläche der Patientenlagerungsvorrichtung 100 zu bestimmen.

Die Bestimmungseinrichtung 16 kann mit der Schnittstelle 12 und der Erfassungseinrichtung 14 gekoppelt sein. In Ausführungsbeispielen kann die Bestimmungseinrichtung 16 einem beliebigen Controller oder Prozessor oder einer programmierbaren Hardwarekomponente entsprechen. Beispielsweise kann die Bestimmungseinrichtung 16 auch als Software realisiert sein, die für eine entsprechende Hardwarekomponente programmiert ist. Insofern kann die Bestimmungseinrichtung 16 als programmierbare Hardware mit entsprechend angepasster Software implementiert sein. Dabei können beliebige Prozessoren, wie Digitale SignalProzessoren (DSPs) oder Grafikprozessoren zum Einsatz kommen. Ausführungsbeispiele sind dabei nicht auf einen bestimmten Typ von Prozessor eingeschränkt. Es sind beliebige Prozessoren oder auch mehrere Prozessoren zur Implementierung der Bestimmungseinrichtung 16 denkbar. Die Fig. 1 illustriert ferner, dass in einigen Ausführungsbeispielen die Bestimmungseinrichtung 16 mit der Erfassungseinrichtung 14 gekoppelt sein kann. In einem solchen Ausführungsbeispiel erfassen beispielsweise die ein oder mehreren Sensoren der Erfassungseinrichtung 14 zumindest dreidimensionale (Teil-) Bilddaten und stellen diese der Bestimmungseinrichtung 16 bereit, die in den Bilddaten die Patientenlagerungsvorrichtung 100, die Lage des zumindest einen Teilsegmentes 120 und der zumindest einen Seitenbegrenzung 110 bestimmt.

In einem Ausführungsbeispiel ist die Bestimmungseinrichtung 16 durch eine Prozessoreinheit implementiert, die mit den 1... n Sensoren verbunden ist, und auf der das beschriebene Verfahren ausgeführt wird. Dabei kann es ferner Kommunikationsverbindungen geben, auch realisiert über die Schnittstelle 12, um die Sensoren mit der Prozessoreinheit zu verbinden und um das Ergebnis des Verfahrens an weitere Systeme, wie Anzeige- oder Dokumentationssysteme, übermitteln zu können.

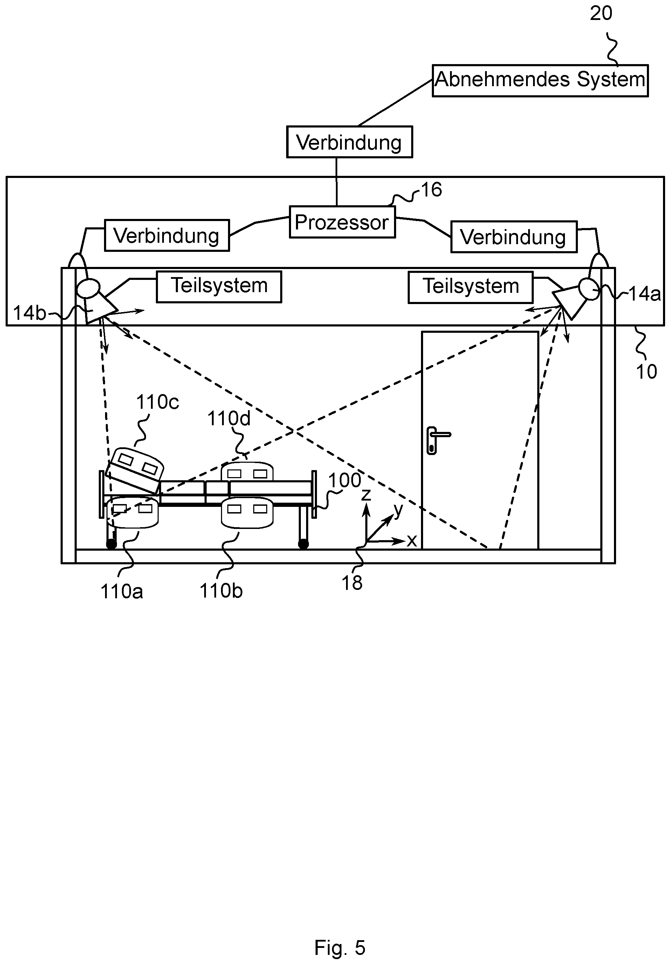

Eine konzeptuelle Darstellung der Vorrichtung 10 ist in Fig. 5 zu sehen. Fig. 5 zeigt ein Ausführungsbeispiel in einem Krankenzimmer. Die Vorrichtung 10 umfasst hier eine Erfassungseinrichtung 14 mit zwei Teilsystemen 14a und 14b zur Erfassung zumindest dreidimensionaler Teilbilddaten aus unterschiedlichen Perspektiven der Szene in dem Krankenzimmer. Die Fig. 5 zeigt darüber hinaus ein konfigurierbares Krankenbett 100 (stellvertretend für eine allgemeine Patientenlagerungsvorrichtung 100) und eine Tür. Die beiden Teilsysteme 14a und 14b der Erfassungseinrichtung 4 sind über eine Kommunikationsverbindung, beispielsweise über Ethernet und Internet-Protokoll (IP) und/oder in einem Netzwerk, mit der Bestimmungseinrichtung 16 gekoppelt, die hier als Prozessoreinheit 16 implementiert ist. Die Fig. 5 zeigt ferner ein kartesisches Koordinatensystem 18, das den nachfolgenden Betrachtungen zugrunde gelegt wird.

Generell kann die Vorrichtung 10 1...n Sensoren umfassen, die jeweils eine Punktemenge bestimmen, die zu einer einzigen dreidimensionalen (Teil-)Bildpunktmenge ergänzt oder zusammengeführt werden können. Wie das Ausführungsbeispiel in der Fig. 5 zeigt, kann die Erfassungseinrichtung 14 mehrere Bildsensoren zur Erfassung von zumindest dreidimensionalen Teilbilddaten umfassen. Die Bestimmungseinrichtung 16 ist dann ausgebildet, um die Daten der mehreren Bildsensoren zu Bilddaten eines zumindest dreidimensionalen Teilbildes der Patientenlagerungsvorrichtung (hier Krankenbett) 100 zusammenzufassen und um die Bestimmung der Lage des zumindest einen Teilsegmentes 120, 120a, 120b, 120c, 120d basierend auf dem (zusammengefassten) Teilbild durchzuführen und basierend auf der Lage des zumindest einen Teilsegmentes 120, 120a, 120b, 120c, 120d die Lage der zumindest einen Seitenbegrenzung 110, 110a, 110b, 110c, 110d zu bestimmen. Die zusammengefassten Bilddaten beinhalten beispielsweise Information über die dreidimensionale Oberfläche der Patientenlagerungsvorrichtung 100 aus den Blickwinkeln der Sensoren. Durch die Zusammenführung der Daten mehrerer Bildsensoren kann ein dreidimensionales (Teil-)Bild des abzubildenden Krankenbettes 100 mit einem höheren Detailgrad erzeugt werden, als mit einem Einzelbild.

Die Bestimmungseinrichtung 16, die in dem Ausführungsbeispiel der Fig. 5 als Prozessoreinheit ausgebildet ist, ist über ein Netzwerk mit den 1..n Sensoren verbunden. Die eigentliche Bestimmung kann dann basierend auf den zusammengeführten Daten durchgeführt werden. Das Netzwerk, das eine Kommunikationsverbindung bereitstellen kann, kann auch dem Weiterleiten von Information über eine bestimmte Bettkonfiguration dienen, beispielsweise zum Zwecke der Dokumentation, Überwachung oder der Darstellung (z.B. auf einem Monitor oder Display). Die Fig. 5 zeigt ein abnehmendes System 20, das ebenfalls über eine Kommunikationsverbindung mit der Bestimmungseinrichtung 16 gekoppelt sein kann, beispielsweise auch über die oben beschriebene Schnittstelle 12. Das abnehmende System 20 nimmt eine Information über die Lage der Seitenbegrenzung 110 ab und verarbeitet diese weiter, beispielsweise zur Dokumentation, Sicherheitsüberwachung, Alarmierung, usw.

Die Darstellung zeigt zwei Sensoren, die die Szene beobachten und die durch eine Kommunikationsverbindung mit der Prozessoreinheit/Recheneinheit 16 verbunden sind, die das beschriebene Verfahren ausführt und selbst wiederum über eine Kommunikationsverbindung mit abnehmenden Systemen verbunden ist. Die Darstellung enthält des Weiteren ein schematisches Bett, wie es auf einer Intensivstation eingesetzt werden könnte. Auf der dem Betrachter zugewandten Seite sind die Seitenbegrenzungen des Bettes tiefgestellt, auf der anderen Seite hochgestellt.

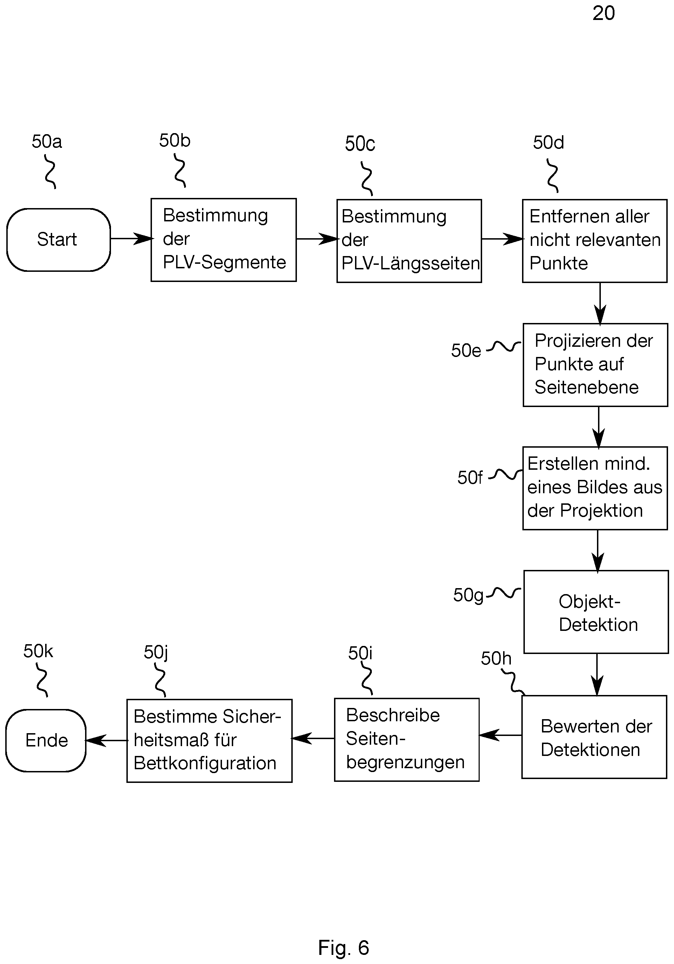

Ein Verfahren, welches auf einer Prozessoreinheit ausführbar ist, ist im Flussdiagramm in Fig. 6 dargestellt. Fig. 6 zeigt ein Ablaufdiagramm zur Bestimmung der Lage der Seitenbegrenzung 110 in einem Ausführungsbeispiel. Als Eingabe zum Startschritt 50a erwartet das Verfahren eine 3D-Punktewolke der PLV 100, für welche der Status der Seitenbegrenzungen ermittelt werden soll. Diese Punktewolke aus der gesamten Punktewolke der Szene auszuschneiden, kann durch Objektdetektions- und Segmentierungsverfahren bewerkstelligt werden. Das Verfahren beginnt damit die Liegefläche der PLV näher zu beschreiben, das heißt, dass nach diesem Ausführungsschritt, Bestimmung der PLV-Segmente 50b, die Lage (Orientierung und Position) sowie die Größe der Liegefläche, ggfs. in Form von mehreren zu beschreibenden Segmenten 120, bekannt ist. Dieser Schritt wird ebenfalls in Dokument DE 10 2015 013 031 beschrieben.

Anschließend folgt die Bestimmung der beiden Längsseiten 50c der PLV. In einigen Ausführungsbeispielen kann die Bestimmungseinrichtung 16 ausgebildet sein, um zwei Längsseiten der Patientenlagerungsvorrichtung 100 zu bestimmen, und basierend auf den Längsseiten aus den Bilddaten Bildpunkte zumindest teilweise auszuschließen, die nicht zu der zumindest einen Seitenbegrenzung 110 der Patientenlagerungsvorrichtung 100 gehören. Entlang der Längsseiten befinden sich, falls vorhanden, die Seitenbegrenzungen 110. Basierend auf den bisher ermittelten Informationen werden nun möglichst alle Punkte entfernt 50d, die nicht zu den Seitenbegrenzungen 110 gehören können. Auch kann es an dieser Stelle nötig sein, die verbliebenden Punkte in zwei Teilmengen zu zerteilen 50e, für jede PLV-Seite eine. Die Bestimmungseinrichtung 16 ist dann ausgebildet, um die Bilddaten ferner auf Bildpunkte einer Längsseite der Patientenlagerungsvorrichtung 100 einzuschränken. Die Bestimmungseinrichtung 16 kann auch ausgebildet sein, um die Bilddaten auf eine Seitenebene der Patientenlagerungsvorrichtung 100 zu projizieren und um ein Projektionsbild zu erhalten.

Die Schritte 50e-50k beziehen sich auf die Erkennung der Seitenbegrenzungen 110 einer PLV-Seite und werden daher für jede Seite separat mit der jeweiligen Punkte-Teilmenge ausgeführt, sofern das Verfahren Aussagen über beide Seiten treffen soll. Die verbliebenden Punkte der gewählten Teilmenge werden dann auf eine Seitenebene der PLV projiziert 50e. Aus der entstehenden Projektion erstellt das Verfahren im vorliegenden Ausführungsbeispiel mindestens ein Bild 50f in welchem anschließend mit Hilfe von Objekt-Detektoren 50g Seitenbegrenzungen gesucht werden. Die Bestimmungseinrichtung 16 ist ausgebildet ist, um in dem Projektionsbild mittels einer Objekt-Detektion Seitenbegrenzungskandidaten zu bestimmen. Die von den Objekt-Detektoren erstellten Detektionen werden im Folgenden bewertet 50h und basierend auf der Bewertung und der Lage der PLV-Segmente 120 wird eine Ausgabe 50i erstellt, die die Seitenbegrenzungen 110 beschreibt. Die Bestimmungseinrichtung 16 ist ausgebildet, um die Seitenbegrenzungskandidaten zu bewerten und um basierend auf den bewerteten Seitenbegrenzungskandidaten die Lage der zumindest einen Seitenbegrenzung 110zu bestimmen.

Im letzten Schritt 50j erfolgt eine automatisierte Bewertung der Sicherheit der Bettkonfiguration basierend auf der Einstellung der Seitenbegrenzungen und der Lagesegmente und daraufhin eine mögliche Alarmierung. Die Bestimmungseinrichtung 16 ist ausgebildet, um basierend auf der Lage der zumindest einen Seitenbegrenzung 110 Sicherheitsinformation über eine Konfiguration der Patientenlagerungsvorrichtung 100 zu bestimmen. Die Bestimmungseinrichtung 16 kann z.B. ausgebildet sein, um die Sicherheitsinformation über eine Speichereinrichtung zu dokumentieren. Die Bestimmungseinrichtung 16 kann auch ausgebildet sein, um die Sicherheitsinformation über eine Darstellungseinrichtung, beispielsweise einen Monitor, ein Display, einen Drucker, etc., auszugeben. Die Bestimmungseinrichtung 16 kann ferner ausgebildet sein, um basierend auf der Sicherheitsinformation eine Alarmierungsinformation, z.B. optisches, haptisches oder akustisches Warnsignal für Pflegepersonal, auszugeben, wenn die Konfiguration der Patientenlagerungsvorrichtung 100 ein Sicherheitsniveau unterschreitet.

Ausführungsbeispiele können eine automatisierte Dokumentation der Stellungen der Seitenbegrenzungen 110 bzw. deren Sicherheit ermöglichen. Es kann eine automatisierte Bewertung der Sicherheit einer Bettkonfiguration zur Verwendung in weiteren, sich anschließenden Algorithmen, oder zur Alarmierung erfolgen. Im Vergleich zu anderen Verfahren, die auf einer Patientenlagerungsvorrichtung 100 beruhen, die selbst in der Lage ist die Stellung der Seitenbegrenzungen zu erkennen und zu kommunizieren, ergeben sich für Ausführungsbeispiele eine leichtere Nachrüstbarkeit. Ausführungsbeispiele stellen damit ein universell einsetzbares System bereit, das nicht an bestimmte Patientenlagerungsvorrichtungen gebunden ist. Ausführungsbeispiele benötigen keine am Bett angebrachte Sensorik, die z.B. durch Reinigungsmaßnahmen beschädigt werden kann oder entsprechend aufwendig und teuer gekapselt werden muss. Falls solche Sensoren vorhanden sind, können diese jedoch in Ausführungsbeispielen mit genutzt werden. Statusdaten können auch bei Einsatz verschiedener PLV in einem Bereich stets standardisiert geliefert werden. Auch kann durch Verarbeitung der Bilddaten auf eine störende Verkabelung im Raum verzichtet werden. Die Lage und Position der Seitenbegrenzungsteile im Raum werden detektierbar und stehen für weitere Verarbeitung zur Verfügung (z.B. für Kollisionswarnung/-detektion).

Im Folgenden werden einige Ausführungsbeispiele im Detail erläutert. Dazu werden insbesondere mögliche Ausführungen für die einzelnen vorgestellten Schritte des Verfahrens vorgestellt. Die Bestimmungseinrichtung kann demnach die verschiedenen Schritte und Detektoren implementieren. In diesem Ausführungsbeispiel wird ein 3D-Sensor verwendet, um die dreidimensionalen Punkte, die die PLV 100 beschreiben, zu erhalten. Als Prozessoreinheit 16 kann ein handelsüblicher Desktopcomputer zum Einsatz kommen. Im weiteren Verlauf wird mindestens eine mögliche Ausführung für jeden in der Fig. 6 skizzierten Schritte näher beschrieben.

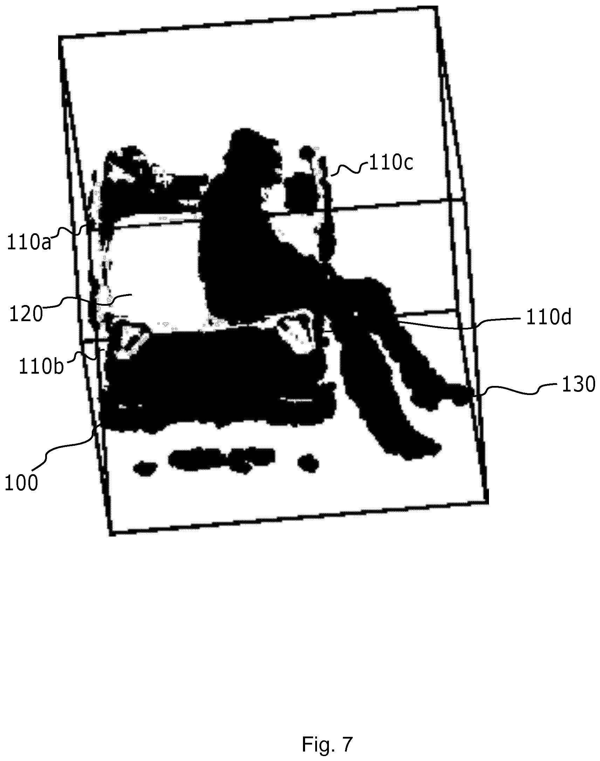

Die Eingabe-Punktewolke für das Verfahren an welcher ein Ausführungsbeispiel demonstriert wird, ist zusammen mit der für die Punktewolke berechneten Oriented-Bounding-Box (OBB, Begrenzungsrahmen) in Fig. 7 zu sehen. Fig. 7 zeigt erfasste Bilddaten mit einem Begrenzungsrahmen in einem Ausführungsbeispiel. Dargestellt ist ein Bett 100 einer Intensivstation, dessen Segmente 120 so eingestellt sind, dass sie horizontal zum Boden ausgerichtet sind. Auf dem Bett befindet sich sitzend ein Patient 130. Das Bett hat Seitenbegrenzungen 110, die pro Bettseite aus zwei Teilen bestehen. Hierbei sind die beiden Seitenbegrenzungen 110 auf der linken Seite des Bettes 100 hochgestellt, auf der rechten Seite ist nur das zur Kopfseite des Bettes 100 zugehörige Bettgitter 110 hochgestellt. Die OBB für die Eingabe-Punktewolke lässt sich als ein Teilergebnis mit den Verfahren bestimmen, wie im Dokument DE 10 2015 013 031 näher erläutert.

Im vorliegenden Ausführungsbeispiel wird die Liegefläche der PLV 100 in Form von gegeneinander verstellbaren Segmenten 120 beschrieben. Diese Segmente können in Form von ihrer Größe, Position und Orientierung mit den in Dokument DE 10 2015 013 031.5 beschriebenen Verfahren bestimmt werden.

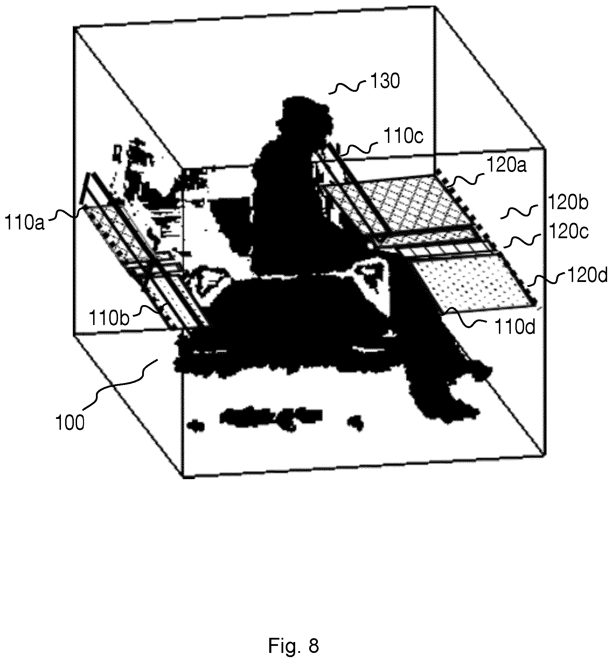

Fig. 8 illustriert die erfassten Bilddaten aus der Fig. 7 mit einem Begrenzungsrahmen und bestimmten Teilsegmenten 120a, 120b, 120c und 120d in dem Ausführungsbeispiel. Ferner sind die vier Seitenbegrenzungen 110a, 110b, 110c und 110d bzw. Bettgitter zu erkennen. Fig. 8 zeigt die in diesem Fall vier berechneten PLV-Segmente 120a, 120b, 120c und 120d in der Eingabe-Punktewolke. Wie weiter zu sehen ist, sind die PLV-Segmente 120a, 120b, 120c und 120d in ihrer Größe nicht korrekt, da die OBB, aufgrund der sitzenden Person 130, zu groß berechnet wurde. Es ist daher in diesem Beispiel nicht möglich, die rechte Seitenbegrenzung 110c, 110d zu bestimmen, in dem man nur in unmittelbarer Nähe der rechten Begrenzung der OBB sucht. Der folgende Schritt korrigiert dies.

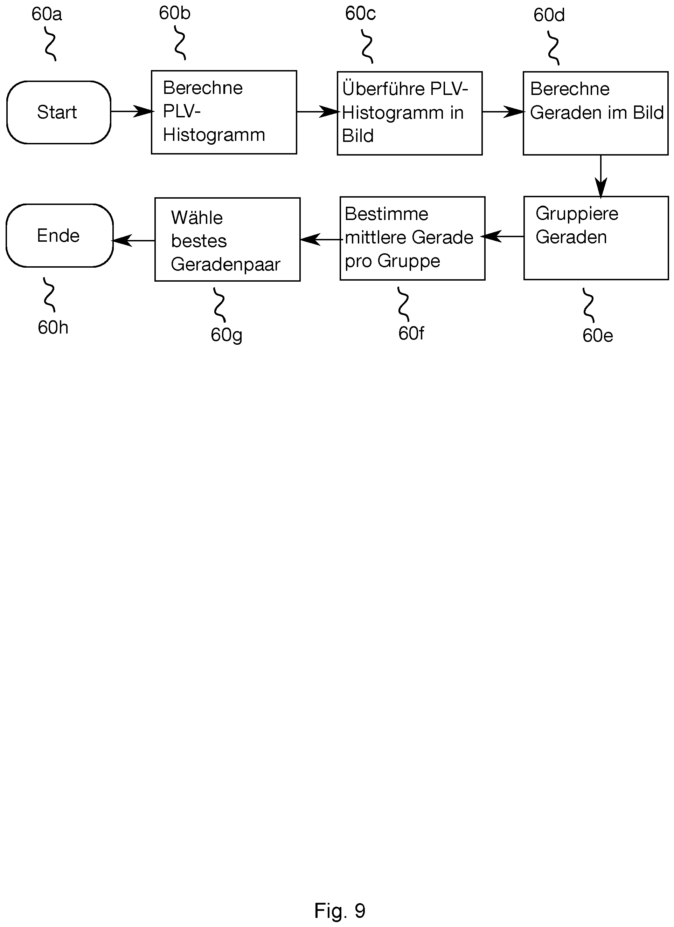

Um die Längsseiten der PLV 100 zu bestimmen an denen die Seitenbegrenzungen 110a-d potentiell angebracht sein können, wird in diesem Ausführungsbeispiel wie in Fig. 9 vorgegangen. Fig. 9 zeigt ein Ablaufdiagramm zur Bestimmung der Seitenbegrenzung 110a-d in dem Ausführungsbeispiel. Die Hauptaufgabe des Verfahrens liegt darin, an den Seiten überstehende Objekte "abzuschneiden". Die Grundidee ist ein Bild aus der Eingabe-Punktewolke zu erstellen, in dem Geraden gesucht werden. Das Geradenpaar, das die Seiten der PLV 100 am besten beschreibt, wird dann ausgewählt und dazu verwendet, die Längsseiten zu bestimmen. In dem betrachteten Ausführungsbeispiel beginnt das Verfahren in Schritt 60a.

Zunächst wird ein Histogramm aus der Eingabe-Punktewolke erstellt 60b, in dem die Punkte, die über einem der die PLV-Segmente 120a-d beschreibenden Rechtecke liegen, auf das jeweilige Rechteck projiziert werden. Anschließend wird pro Segment 120a-d (bzw. Rechteck) ein zweidimensionales Histogramm erstellt, so dass die jeweils projizierten Punkte in Klassen aufgeteilt und gezählt werden. Die Histogramme werden nun zu einem einzigen Histogramm verknüpft, wobei die Reihenfolge der einzelnen Histogramme (von Fußseite zu Kopfseite) erhalten bleibt. Es ist hierbei anzumerken, dass das Verfahren auf die PLV-Segmente 120a-d angewiesen ist, um die Histogramme wahrheitsgetreu zu erstellen. Wäre bspw. das Kopfsegment der PLV 100 schräggestellt, wäre eine Projektion der Punkte auf die Fußbodenebene nicht korrekt, da die Punkte dann in der Projektion einen verhältnismäßig deutlich geringeren Bereich abdecken würden, als wenn das Kopfsegment parallel zum Boden orientiert wäre. Es ergibt sich daher ein robusteres Verfahren, da die PLV-Segmente 120a-d verwendet werden.

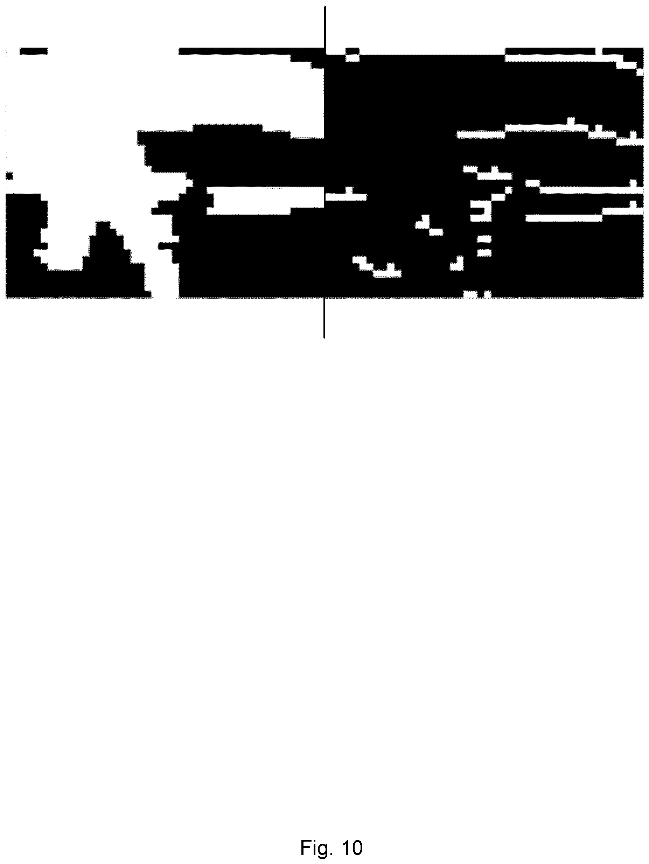

Im nächsten Schritt 60c erzeugt das Verfahren aus dem verknüpften Histogramm ein Bild, das für das weitere Verfahren verwendet wird. Dies geschieht, in dem ein Schwellwert auf das Histogramm angewendet wird und alle Histogramm-Klassen, deren Wert über dem Schwellwert liegt, einen wei-ßen Pixel und alle anderen Klassen einen schwarzen Pixel erzeugen. Anschließend werden Löcher im Bild gefüllt (siehe (Soille, 1999)) und mit einem Kantendetektor ein Kantenbild erstellt (hierfür gibt es in der Literatur eine Vielzahl von Algorithmen, z.B. (Canny, 1986)). Für beide Schritte ist ein Beispiel in Fig. 10 gegeben. Die linke Seite zeigt das Histogramm-Bild nach dem Füllen der Löcher und die rechte Seite das daraus erstellte Kantenbild, jeweils für das vorliegende Ausführungsbeispiel. Es lässt sich erkennen, dass die beiden Bettseiten im Wesentlichen auf einer Geraden liegen, die insbesondere durch die Beine des Patienten 130 unterbrochen wird.

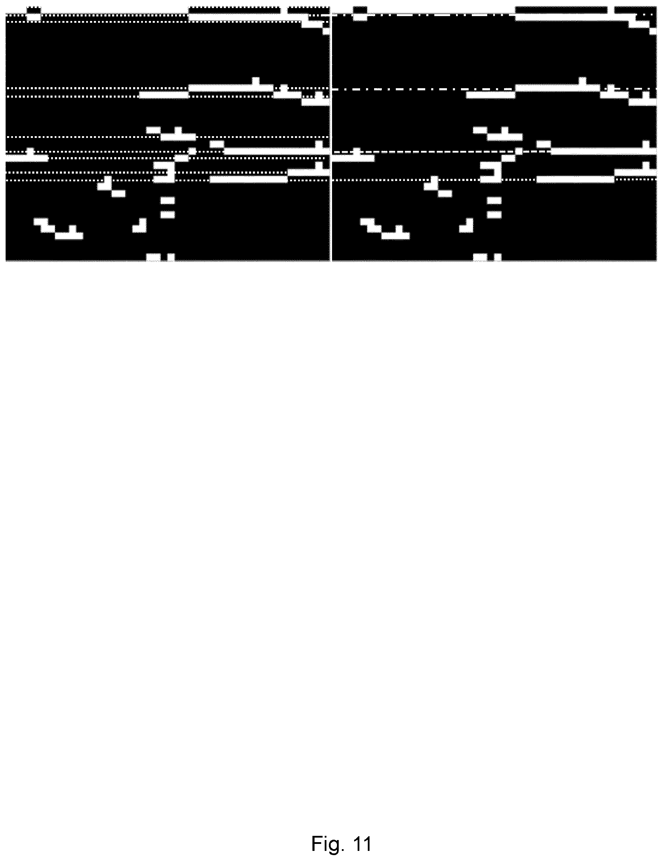

Das Verfahren fährt fort, indem in dem erzeugten Kantenbild Geraden 60d gesucht werden. Eine Möglichkeit um dies zu bewerkstelligen ist die sogenannte Hough-Transformation (Duda & Hart, 1972). Für das hier verwendete Ausführungsbeispiel sind die Geraden in Fig. 11 links dargestellt. Man beachte, dass sich die Geraden teilweise so ähneln, dass sie nahezu exakt übereinander liegen und daher in der Fig. 11 nicht einzeln erkannt werden können.

Oftmals findet die Hough-Transformation eine Vielzahl von Geraden, die jeweils dieselbe Bettseite beschreiben. Aus diesem Grund werden die Geraden gruppiert, wobei jede Gruppe später zu einer Geraden zusammengefasst wird 60e. Das Gruppieren 60e geschieht dadurch, dass ein Abstandsmaß zwischen zwei Geraden definiert wird und darauf basierend ein Graph erstellt wird. Eine Gerade wird in diesem Graphen als Knoten repräsentiert und zwei Knoten sind durch eine Kante verbunden, wenn das jeweilige Abstandsmaß beider repräsentierter Geraden kleiner als ein definierter Schwellwert war. Durch das Identifizieren von Cliquen im erzeugten Graphen (z.B. mit Hilfe des Bron-Kerbosch Algorithmus (Bron & Kerbosch, 1973)) werden die Geraden nun gruppiert, wobei eine Clique einer Gruppe entspricht. Das Abstandsmaß basiert auf den Steigungen der Geraden und den Punkten, in denen sie den linken Bildrand schneiden. In der Fig. 11 sind links alle gefundenen Geraden des Verfahrens dargestellt, und rechts die verbliebenden mittleren Geraden.

Pro gefundener Clique wird im nächsten Schritt 60f eine repräsentative, gemittelte Gerade bestimmt. Dies geschieht unter Berücksichtigung des "Geradengewichts" der einzelnen Geraden der Gruppe, wobei das Geradengewicht beispielsweise definiert werden kann als die Anzahl weißer Pixel, die nah genug an der jeweiligen Gerade liegen. Die Fig. 11 zeigt rechts eine mittlere Gerade pro identifizierter Clique, in diesem Ausführungsbeispiel vier. Abschließend versucht das Verfahren aus den verbliebenden Geraden das beste Paar zu finden 60g. Dies geschieht unter Berücksichtigung der Steigungen der Geraden (Geraden eines Paares sollten möglichst eine ähnliche Steigung haben) sowie dem Abstand der Geraden, wobei bei letzterem Vorwissen über die typische Breite einer PLV 100 mit einbezogen werden kann. Im vorliegenden Ausführungsbeispiel werden so die Geraden oben und unten ausgewählt.

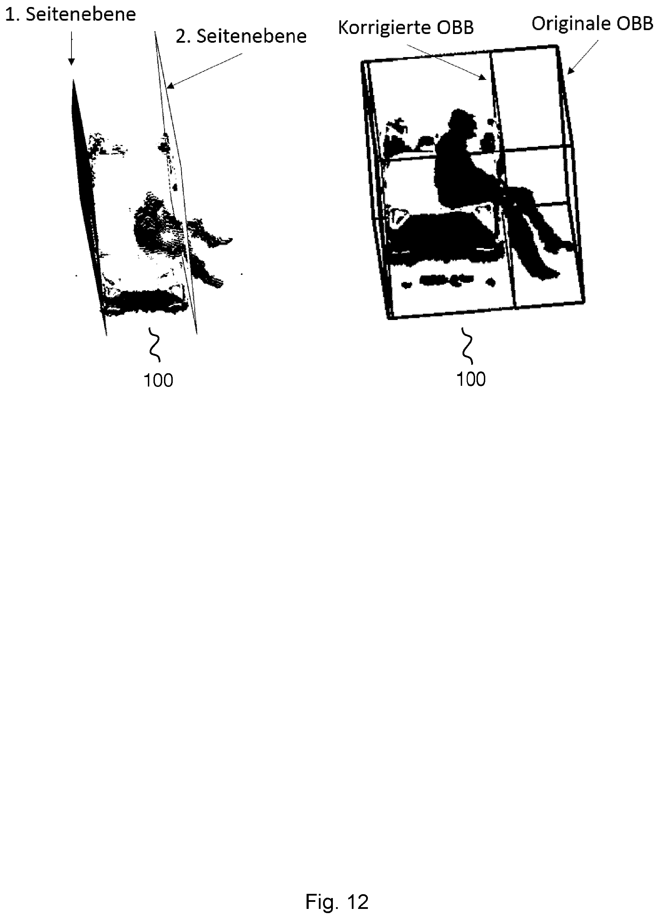

Fig. 12 zeigt basierend auf Seitenebenen gewonnene Begrenzungsrahmen in dem Ausführungsbeispiel. In Fig. 12 sind links die aus den beiden Geraden gewonnenen Ebenen, die die Längsseiten der PLV 100 repräsentieren, dargestellt. Rechts ist die daraufhin korrigierte OBB zu sehen und die ursprüngliche, falsche oder zu große, OBB. Sobald die Längsseiten der PLV 100 bestimmt sind, werden möglichst alle Punkte aus der Punktewolke entfernt, die nicht zu den Seitenbegrenzungen gehören können. Da die Seitenbegrenzungen entlang der Längsseiten der PLV 100 verlaufen, werden hier alle Punkte verworfen, die zwischen den beiden Seitenebenen liegen und um ein Mindestmaß (z.B. 10 cm) von diesen entfernt liegen sowie die Punkte, die nicht zwischen beiden Seitenebenen und mindestens um ein zweites Mindestmaß (z.B. 10 cm) von beiden entfernt liegen.

In Fig. 12 würden in diesem Ausführungsbeispiel also alle Punkte erhalten bleiben, die maximal 10 cm von einer der beiden Ebenen entfernt liegen. Diese würden die Längsseiten des Bettes mit den Seitenbegrenzungen sowie einen Teil der sitzenden Person beinhalten.

Die folgenden Schritte beziehen sich auf das Bestimmen der Seitenbegrenzung 110 einer PLV-Seite. In diesem Ausführungsbeispiel wird im Folgenden die linke Seite der PLV 100 betrachtet. Das Aufteilen der verbliebenden Punkte in Teilmengen, je eine Menge für jede PLV-Seite, erfolgt am Abstand der Punkte zu den beiden Seitenebenen. Punkte, die näher an der linken Seitenebene sind, werden der Teilmenge für die linke PLV-Seite zugeordnet, alle anderen Punkte der Teilmenge für die rechte PLV-Seite. Das Projizieren, vgl. 50e in Fig. 6, auf die linke Seitenebene selbst, ist durch bekannte Verfahren der Mathematik zu bewerkstelligen.

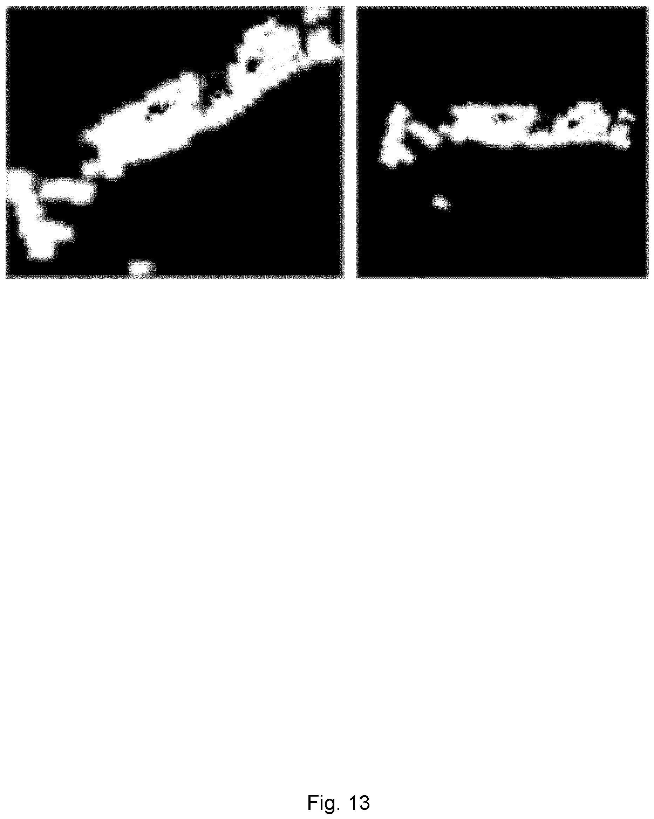

Ähnlich wie oben beschrieben wird auch hier aus den projizierten Punkten ein Bild erstellt. Das heißt, dass wiederum ein Histogramm der Punkte berechnet und jede Histogramm-Klasse als Pixel des Bildes aufgefasst wird. Anschließend lässt sich unter Verwendung eines Schwellwertes ein Schwarz-Weiß-Bild erstellen. Filter, insbesondere Gauß-Filter, können dann auf das Bild angewendet werden, um z.B. Effekte, die durch Rauschen entstehen, zu reduzieren. Das Ergebnis dieses Ausführungsbeispiels mit Verwendung eines Gauß-Filters ist in Fig. 13 links zu sehen. Fig. 13 zeigt eine Illustration eines Projektionsbildes in dem Ausführungsbeispiel unter Kenntnis einer Orientierung einer Liegefläche einer Patientenlagerungsvorrichtung 100. Die Fig. 13 stellt links ein erstelltes Bild der projizierten Punkte dar und rechts dasselbe Bild mit Kenntnis der Orientierung der Liegefläche in rotierter Darstellung.

Die beiden Teile der Seitenbegrenzung lassen sich erkennen, sowie unter anderem auch die Fußplatte. Im nächsten Schritt sollen die Seitenbegrenzung(en) 110 gesucht werden. Um diesen Schritt möglichst robust zu machen, kann es ratsam sein, die Varianz in den Erscheinungen der Seitenbegrenzungen 110 so gering wie möglich zu halten. An dieser Stelle lässt sich das Wissen über die Lage der PLV-Segmente 120a-d verwenden, indem das Bild segmentweise so gedreht wird, dass die PLV-Segmente 120a-d parallel zur Horizontalachse des Bildes verlaufen. Damit wird eine Invarianz gegenüber Verstellungen der PLV-Segmente 120a-d erreicht, da die Seitenbegrenzungen 110 nun ebenfalls parallel zur Horizontalachse des Bildes ausgerichtet sind, was den Objekt-Detektoren zusätzliche Annahmen ermöglicht.

Obiges ist wahr, wenn die Seitenbegrenzungen 110 an den PLV-Segmenten 120a-d befestigt sind. Es kommt jedoch auch vor, dass sie stattdessen an der PLV-Längsachse montiert werden. Um die Lage, und insbesondere die Orientierung der Längsachse zu ermitteln, wäre beispielsweise folgendes Verfahren möglich:

1) Entfernen der Punkte, die lediglich maximal D cm vom Fußboden entfernt liegen (diese Punkte können nicht zur PLV-Längsachse gehören, denn die PLVs 100 müssen in der Regel eine gewisse Mindestarbeitshöhe für das medizinische Personal garantieren),

2) Entfernen der Punkte, die oberhalb der Liegefläche liegen (hier werden die ermittelten PLV-Segmente 120a-d benötigt),

3) Entfernen der Punkte, die wahrscheinlich zur Matratze gehören (bspw. indem Punkte, die F cm unter den jeweiligen PLV-Segmenten 120a-d liegen, entfernt werden),

4) Berechnen, basierend auf den verbleibenden Punkten, einer Hauptkomponentenanalyse (auch engl. Principal Component Analysis, PCA) (Jolliffe, 2002) und wählen der Hauptachse als PLV-Längsachse.

Ist die Orientierung der PLV-Längsachse somit bekannt, lässt sich das Bild auch so rotieren, dass diese Längsachse parallel zur Horizontalachse des Bildes verläuft. Ist nicht bekannt zu welchem Typ die PLV 100 gehört, können mehrere rotierte Bilder (rotiert unter Berücksichtigung der PLV-Segmente 120a-d, rotiert anhand der Längsachse, etc.) erstellt werden und in dieser Menge von Bildem nach Seitenbegrenzungen 110 gesucht werden. Die Fig. 13 zeigt rechts das erstellte Bild des Beispiels rotiert, so dass die PLV-Segmente 120a-d parallel zur Horizontalachse des Bildes sind.

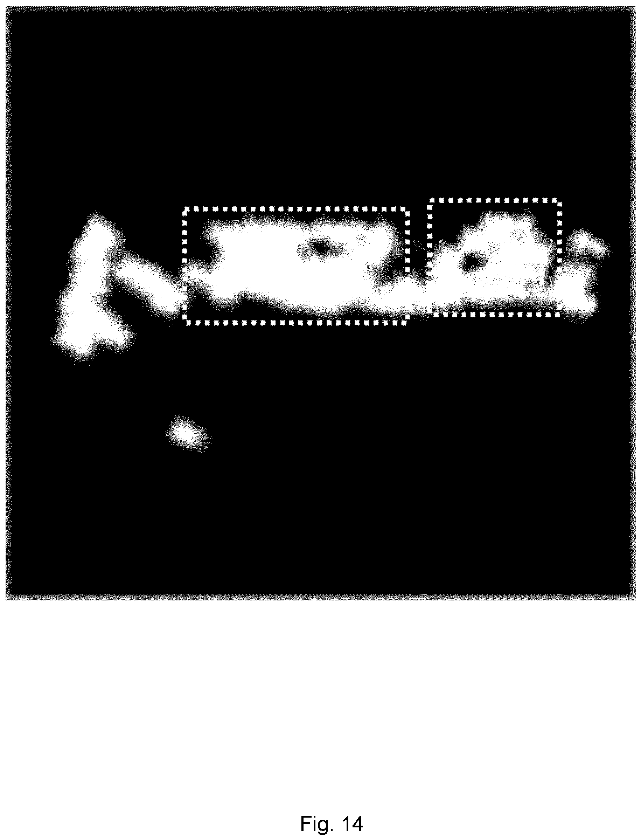

In dem vorliegenden Ausführungsbeispiel wird in dem rotierten Bild (Fig. 13 rechts) nach Seitenbegrenzungen gesucht, indem trainierte Objekt-Detektoren verwendet werden. Nach dem Viola-Jones-Algorithmus (Viola & Jones, 2001) wird unter Verwendung von HOG-Merkmalen (Dalal & Triggs, 2005, engl. Histograms of Oriented Gradients, Histogramme orientierter Gradienten) ein Objekt-Detektor trainiert, der in der Lage ist, Seitenbegrenzungen im Bild zu erkennen. HOG ist ein Merkmal, das auf Histogrammen von Gradienten und deren Orientierung in verschiedenen Bildbereichen basiert. Das Ergebnis der Suche des Detektors ist in Fig. 14 dargestellt. Fig. 14 zeigt von einem Objekt-Detektor identifizierte Teilsegmente und Seitenbegrenzungen in einem Ausführungsbeispiel.

Der Detektor hat die beiden Teile der linken Seitenbegrenzung erfolgreich erkannt und lokalisiert (erkennbar an den gestrichelten Rechtecken in Fig. 14). Es sei angemerkt, dass Objekt-Detektoren nach dem Viola-Jones-Algorithmus nicht die einzige Möglichkeit darstellen, die Seitenbegrenzungen zu lokalisieren. Ebenso wäre es z.B. möglich, das Bild mit einem "Sliding Window"-Ansatz abzusuchen. Dabei wird ein Rechteck ("window") in verschiedenen Größen über das Bild verschoben ("sliding") und für jedes so gewählte Rechteck ein Merkmalsvektor bestimmt, auf dessen Basis ein Klassifikator entscheidet, ob sich in dem Rechteck eine Seitenbegrenzung befindet oder nicht. Als Merkmalsvektor ließen sich beispielsweise das oben bereits erwähnte HOG (Dalal & Triggs, 2005), Local Binary Patterns oder Haar-Merkmale verwenden. Auch eine Kombination dieser Merkmale wäre möglich. Als Klassifikatoren könnten eine Support Vector Machine oder ein Random Forest Klassifikator zum Einsatz kommen. Weitere Details zu diesen Detektoren und Algorithmen finden sich beispielsweise in

Ojala, T., M. Pietikainen, and T. Maenpaa, "Multiresolution Gray-scale and Rotation Invariant Texture Classification With Local Binary Patterns". IEEE Transactions on Pattern Analysis and Machine Intelligence. Volume 24, No. 7 July 2002, pp. 971-987, Viola, P., and M. J. Jones, "Rapid Object Detection using a Boosted Cascade of Simple Features". Proceedings of the 2001 IEEE Computer Society Conference. Volume 1, 15 April 2001, pp. I-511-I-518, Cortes, Corinna, and Vladimir Vapnik. "Support-vector networks." Machine learning 20.3 (1995): 273-297, und
Breiman L., Random forests. In Machine Learning, Seiten 5-32, 2001.

Es kann durchaus passieren, dass die Objekt-Detektoren mehrere, u.U. falsche, Detektionen machen. Es ist daher ein nachgelagerter Schritt angebracht, der die erstellte Menge von Detektionen bewertet, vgl. 50h in Fig. 6. In diesem Ausführungsbeispiel werden verschiedene Gegebenheiten ausgenutzt, um die Detektionen zu bewerten und auszusortieren. So können z.B. nicht mehrere Seitenbegrenzungen übereinander (gesehen vom Fußboden zur Decke) angeordnet sein. Dabei kann ausgenutzt werden, dass viele bekannte Verfahren der Objekt-Detektion, wie auch der Viola-Jones-Algorithmus, eine Konfidenz für jede Detektion angeben. So lässt sich bspw. von übereinander liegenden Detektionen nur die Detektion mit der höchsten Konfidenz behalten. Alternativ oder ergänzend können Detektionen, die unter der Liegefläche angeordnet sind, bevorzugt werden. Der Hintergedanke hierbei ist, dass auf der Liegefläche störende Objekte liegen können (wie z.B. Kissen), die ggfs. zu falschen Detektionen führen. Findet man jedoch bereits eine tiefgestellte Seitenbegrenzung in diesem PLV-Abschnitt, können die falschen, auf das Kissen zurückzuführende, Detektionen aussortiert werden. Die Seitenbegrenzungen 110 der PLV 100 bestehen aus einem oder zwei Teilen pro Seite. Daher lässt sich ferner die Anzahl der Detektionen pro Seite auf Basis der Konfidenzmaße auf maximal zwei beschränken.

Das Ergebnis des Verfahrens oder die Beschreibung der Seitenbegrenzungen kann vielfach ausgestaltet sein. Zunächst kann die Ausbildung der Seitenbegrenzung beschrieben werden, d.h. anhand der Detektionen wird entschieden, ob es sich um eine einteilige oder zweiteilige Seitenbegrenzung (natürlich wieder pro Seite) handelt. Jedes Teil kann nun weiter in seiner Einstellung beschrieben werden, z.B.

1) durch ein Polygon im dreidimensionalen Raum,
2) durch einen Quader im dreidimensionalen Raum,
3) durch seine mittlere Höhe über (bzw. unter) der Liegefläche oder PLV-Längsachse im Abschnitt des betrachteten Teils, und
4) durch eine Zahl zwischen x (z.B. 0) und y (z.B. 10), wobei x die niedrigste Stellung und y die höchste Stellung des Teils angibt. Die Stellungen x und y könnten z.B. durch den Typ der PLV bekannt sein.

Man beachte, dass die Ausgaben, die eine Aussage über die Höhe des Teils der Seitenbegrenzung 110 machen, einen Bezugspunkt benötigen. Hier ist insbesondere die Liegefläche der PLV 100 sinnvoll, denn die Höhe der Seitenbegrenzung über der Liegefläche entscheidet über die Wirkung der Begrenzung selbst. Wie bereits oben erwähnt kann zumindest in manchen Ausführungsbeispielen die Bestimmungseinrichtung 16 ausgebildet sein, um basierend auf der Sicherheitsinformation oder auch basierend auf einem Sicherheitsmaß eine Alarmierungsinformation auszugeben, wenn die Konfiguration der Patientenlagerungsvorrichtung 100 ein Sicherheitsniveau unterschreitet. Z.B. kann das Sicherheitsniveau der Patientenlagerungsvorrichtung 100 auf einer relativen Lage des zumindest einen Teilsegmentes 120 zu der zumindest einen Seitenbegrenzung 110 basieren.

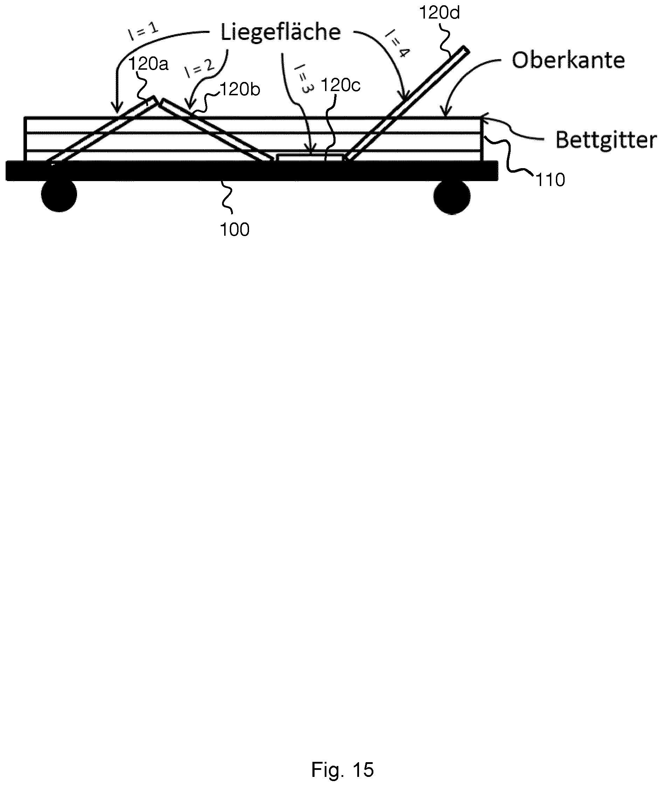

Wie in der Fig. 6 in Schritt 50j beschrieben kann das Sicherheitsmaß für die Bettkonfiguration vor dem Hintergrund der erkannten Einstellungen der Seitenbegrenzungen 110 und Lagesegmente 120 der PLV 100 bestimmt und interpretiert werden, um eine Aussage über die Sicherheit der aktuellen PLV-Konfiguration für den Patienten zu machen. In der Fig. 15 sind die vier Liegeflächen (l= 1, 2, 3, 4) über den jeweiligen Teilsegmenten 120a-d angeordnet. Fig. 15 illustriert mögliche Konfigurationen eines Krankenbettes mit Bettgittern 110 in dem Ausführungsbeispiel. Mit dem hier vorliegenden Verfahren kann in manchen Ausführungsbeispielen diese Aussage wesentlich fundierter getroffen werden, als mit einer einfachen binären (hochgestellt, tiefgestellt) Erkennung. Zur Erläuterung betrachte man bspw. die in Fig. 15 dargestellte PLV-Konfiguration. Das Bettgitter 110 ist hier hochgestellt, jedoch ist die Konfiguration potentiell dennoch gefährlich - insbesondere durch das angehobene Kopfsegment (I=4) der Liegefläche ist es einem Patienten hier trotz des Bettgitters 110 vereinfacht möglich die PLV 100 zu verlassen. Es besteht eine gewisse Sturzgefahr.

Basierend auf der bekannten Lage der Lagesegmente 120 und der Bettgitter 110 und damit deren Relation zueinander ließe sich eine derartige Situation identifizieren. Im Folgenden sollen dafür einige Möglichkeiten aufgezeigt werden.

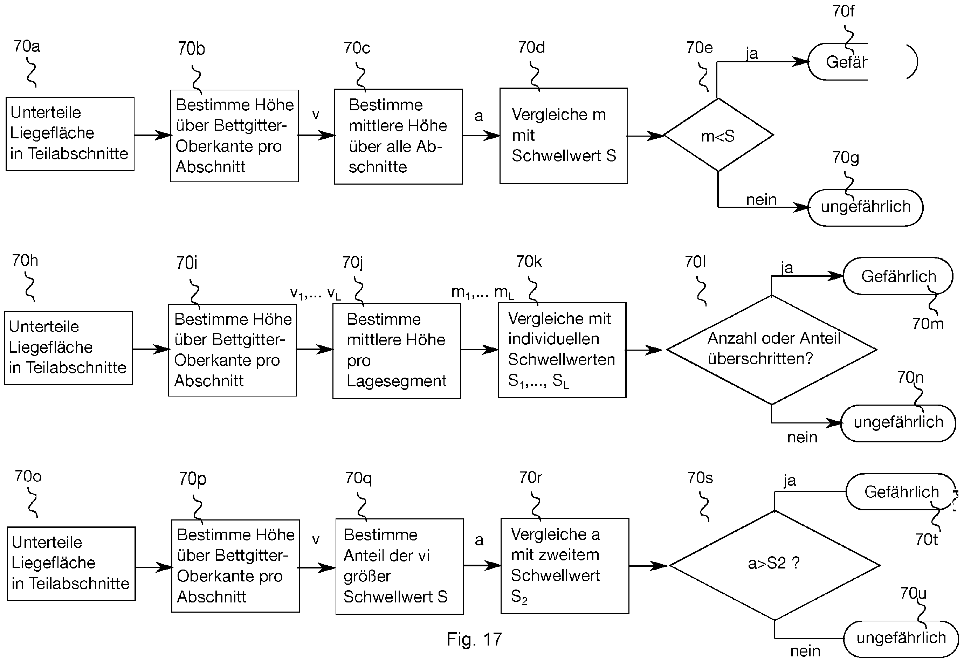

Dabei wird auf die Fig. 17 verwiesen, die verschiedene Ausführungsbeispiele von Verfahren zur Bestimmung einer Sicherheitsinformation zeigen. Fig. 17 stellt "Sicherheitsmaß-Flowcharts" in Form von Flussdiagrammen dar. In einigen Ausführungsbeispielen wird demnach die mittlere Höhe über der Liegefläche bestimmt. Das Sicherheitsniveau kann ein Maß einer mittleren Höhe der Seitenbegrenzung 110 über einer Liegefläche über dem zumindest einen Teilsegment 120 darstellen. Beispielsweise wird zunächst die Liegefläche in Teilabschnitte unterteilt 70a (vgl. Fig. 17 oberes Flussdiagramm). Beispielsweise könnte die Liegefläche in 5cm lange Stücke zerlegt werden, so dass für eine Liegefläche von 2m Länge 40 Teilabschnitte erstellt würden. Anschließend wird für jeden Teilabschnitt die Höhe über bzw. unter der Bettgitter-Oberkante bestimmt 70b, was hier einen Vektor

$$v = \begin{matrix} v_1 \\ \dots \\ v_{40} \end{matrix}$$

ergäbe. Dann wird die mittlere Höhe über der Liegefläche über $m = \frac{1}{40}\sum_{i=1}^{40} v_i$ errechnet, 70c. Abschließend wird m mit einem Schwellwert S verglichen 70d. Die $v_i$ geben die Höhe der Liegeflächenteilabschnitte über der Bettgitter-Oberkante an, d.h. sie sind positiv, wenn der Abschnitt über der Kante liegt und sonst negativ. Große $v_i$ bedeuten dann Gefahr, was im Schritt 70e durch eine Überschreitung des Schwellenwertes S reflektiert wird. Ist die mittlere Höhe m kleiner als S wird die Situation als gefährlich eingestuft 70e-f, andernfalls nicht 70e-g. Soll das Verfahren ein kontinuierliches Maß für die Sicherheit ausgeben, wäre M=m-S eine Möglichkeit.

In einigen Ausführungsbeispielen kann auch eine mittlere Höhe pro Lagesegment 120 bestimmt werden, vgl. 70h, 70i im mittleren Diagramm in Fig. 17. Eine verfeinernde Möglichkeit wäre, die Teilabschnitte v nach Lagesegmenten aufzuteilen, so dass man für jedes Lagesegment einen Teilabschnittsvektor erhält. In diesem

Fall also $v_1,\dots,v_{L=4}$ mit

$$v_i = \begin{matrix} v_{i1} \\ \dots \\ v_{in} \end{matrix}$$

Dann kann für jedes Lagesegment individuell eine mittlere Höhe analog zu oben berechnet werden, 70j, so dass man $m_1,\dots,m_L$ erhält. Diese separaten mittleren Höhen pro Lagesegment können nun mit individuellen Schwellwerten $S_1,\dots,S_4$ verglichen werden. Vergleicht man nun die mittleren Höhen mit ihren entsprechenden Schwellwerten $m_i < S_i$, 70k, so lässt sich eine Aussage über die Sicherheit machen, in dem die Anzahl oder der Anteil der positiv ausgehenden Vergleiche herangezogen wird 70l, 70m, 70n. Dieses Vorgehen könnte es bspw. ermöglichen einen kleinen Schwellwert für das Kopfsegment zu definieren, so dass im Zweifel früher alarmiert wird, wenn ein Sturz mit dem Kopf voran droht.

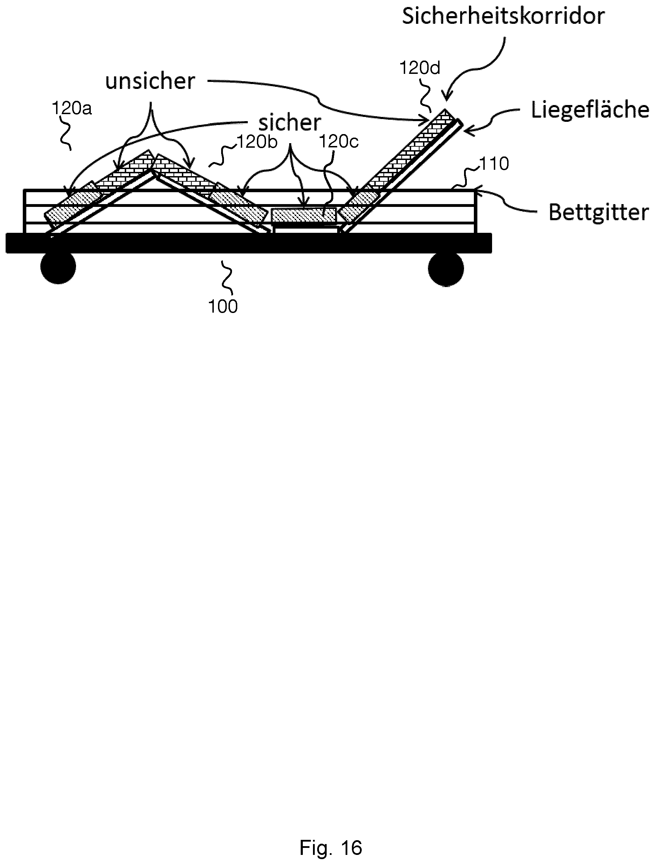

In einem weiteren Ausführungsbeispiel ließe sich der Anteil der sicheren Bereiche ermitteln, vgl. Fig. 17 Flussdiagramm unten, 70o, 70p. Anstatt mittlere Höhen zu berechnen, kann man die Höhen der Bettgitter-Oberkante v über der Liegefläche auch direkt mit einem oder mehreren Schwellwerten S vergleichen, 70q. Somit wird durch S im Prinzip ein Sicherheitskorridor über der Liegefläche definiert. Befindet sich die Bettgitter-Oberkante unterhalb dieses Sicherheitskorridors werden entsprechende Bereiche als unsicher markiert. Zur Verdeutlichung sei hier auf Fig. 16 hingewiesen. Fig. 16 zeigt potentielle Bewertungen in einem Ausführungsbeispiel. Fig. 16 illustriert eine PLV 100, Teilsegmente 120a-d und ein Bettgitter 110. Den Anteil der unsicheren Bereiche a kann man nun mit einem zweiten Schwellwert $S_2$ vergleichen vgl. 70r, 70s, um die Sicherheit der Konfiguration zu bewerten und ggfs. zu alarmieren.

Hauptsächlich unterscheiden sich die oben genannten Möglichkeiten dahingehend, wie die Teilabschnitte v gebildet und ausgewertet werden. Die Flussdiagramme in der Fig. 7 zeigen nur eine Auswertung in "gefährlich" und "ungefährlich", aber in jedem Fall lässt sich auch eine Zahl angeben, die den Grad der Gefahr kontinuierlich angibt (in der Regel der Abstand zum entsprechenden Schwellwert). Nachfolgend an diese Auswertung kann dann z.B. ein Alarmierungsschritt erfolgen.

Ausführungsbeispiele können eine automatisierte Dokumentation der Stellungen der Seitenbegrenzungen 110 bzw. der Sicherheit ermöglichen. In manchen Ausführungsbeispielen kann ein Maß für die Sicherheit einer Bettkonfiguration auf Basis der Stellung der Seitenbegrenzungen 110 und der Teilsegmentlagen 120 bestimmt werden und in einem Dokumentationssystem abgelegt werden. Ein Dokumentationssystem ist typischerweise als Datenbank ausgebildet, in der Informationen zur Sicherheit einer Bettkonfiguration abgelegt werden können. Diese können verschiedenen Datenbankmodellen folgen, wie etwa dem hierarchischen, relationalen, objektorientierten oder dokumentorientierten. Im medizinischen Umfeld sind diese Datenbanken bspw. in klinische Informationssysteme eingebettet.

In einigen weiteren Ausführungsbeispielen kann basierend auf der Sicherheitsinformation eine Alarmierung erfolgen. Das bestimmte Maß für die Sicherheit einer Bettkonfiguration auf Basis der Stellung der Seitenbegrenzungen 110 und der Teilsegmentlagen 120 kann dafür verwendet werden, um bei entsprechender Indizierung automatisch zu alarmieren. Ergibt ein Verfahren in einem Ausführungsbeispiel beispielsweise ein Maß M für die Sicherheit, das kleiner wird je unsicherer eine Bettkonfiguration ist, so ließe sich vom Pflegepersonal ein Schwellenwert S einstellen, bei dessen Unterschreitung alarmiert wird.

In weiteren Ausführungsbeispielen kann ein Anzeigesystem implementiert sein. Die Vorrichtung 10 könnte das errechnete Maß für die Sicherheit einer Bettkonfiguration an ein entferntes Anzeigesystem übermitteln. Zusätzlich ließen sich die Informationen, auf denen das Maß basiert, also die Stellung der Seitenbegrenzungen 110 und die Teilsegmentlagen der Liegefläche, übertragen. Auf diese Weise könnte das medizinische Personal die Einstellung aus der Ferne überprüfen, was zum Beispiel in Hygiene-kritischen Bereichen sinnvoll sein könnte.

Beispiele für solche Anzeigesysteme wären

klinische Informationssysteme,
stationäre Anzeigeeinheiten, wie einfache Bildschirme, Monitore und Displays, und
mobile Anzeigegeräte, wie Smartphones oder Tablets, etc.

Die anzuzeigenden Daten könnten z.B. über ein Netzwerk oder eine Website zur Verfügung stehen, in einer Datenbank abgelegt worden sein oder direkt (z.B. via HDMI, High Definition Multimedia Interface) an eine Anzeigeeinheit weitergegeben werden. Auch wäre eine durch die Alarmierung veranlasste Anzeige möglich. Schlägt das System beispielsweise, wie oben erläutert, Alarm, so könnten die oben genannten Informationen an die oben genannten Anzeigesysteme erst dann übertragen werden.

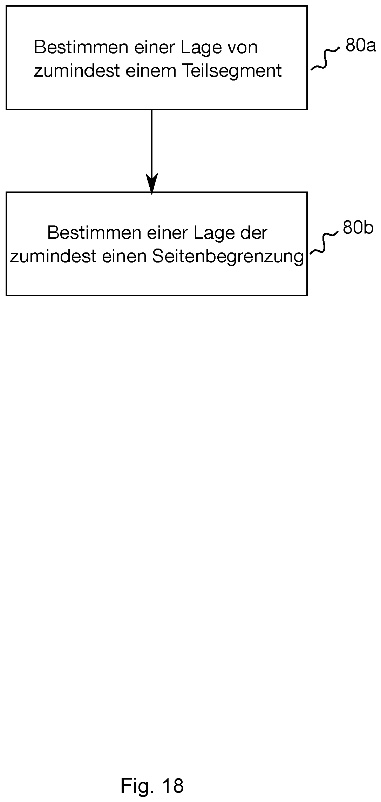

Fig. 18 zeigt ein Blockschaltbild eines Ablaufdiagramms eines Ausführungsbeispiels eines Verfahrens zur Bestimmung einer Seitenebene. Das Verfahren zur Erfassung von optischen Bilddaten einer Patientenlagerungsvorrichtung 100 und zur Bestimmung einer Lage von zumindest einer Seitenbegrenzung 110 der Patientenlagerungsvorrichtung 100 basierend auf den Bilddaten umfasst ein Bestimmen 80a einer Lage von zumindest einem Teilsegment 120 der Patientenlagerungsvorrichtung 100 basierend auf den Bilddaten. Das Verfahren umfasst ferner ein Bestimmen 80b der Lage der zumindest einen Seitenbegrenzung 110 der Patientenlagerungsvorrichtung 100 basierend auf der Lage des zumindest einen Teilsegmentes 120.

Ein weiteres Ausführungsbeispiel ist ein Programm mit einem Programmcode zur Durchführung eines der hierin beschriebenen Verfahren, wenn der Programmcode auf einem Computer, einem Prozessor oder einer programmierbaren Hardwarekomponente ausgeführt wird.

Die in der vorstehenden Beschreibung, den Ansprüchen und den Zeichnungen offenbarten Merkmale können sowohl einzeln als auch in beliebiger Kombination für die Verwirklichung von Ausführungsbeispielen in ihren verschiedenen Ausgestaltungen von Bedeutung sein und - soweit sich nicht aus der Beschreibung etwas anderes ergibt - beliebig miteinander kombiniert werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikro-prozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß einem Ausführungsbeispiel kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch ein Schreiben in eine oder mehrere Speicherstellen eine Aktion bewirken, veranlassen oder durchführen sowie andere Geräte, Maschinen und Komponenten ansteuern.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

**Patentansprüche**

1. Vorrichtung (10) zur Erfassung von optischen Bilddaten einer Patientenlagerungsvorrichtung (100) und zur Bestimmung einer Lage von zumindest einer Seitenbegrenzung (110) der Patientenlagerungsvorrichtung (100) basierend auf den Bilddaten, wobei die Vorrichtung (10) eine Bestimmungseinrichtung (16) umfasst, die zur Bestimmung zunächst einer Lage von zumindest einem Teilsegment (120) der Patientenlagerungsvorrichtung (100) basierend auf den Bilddaten ausgebildet ist, und wobei die Vorrichtung (10) ausgebildet ist, um basierend auf der Lage des zumindest einen Teilsegmentes (120) die Lage der zumindest einen Seitenbegrenzung (110) der Patientenlagerungsvorrichtung (100) zu bestimmen und wobei die Bestimmungseinrichtung (16) ausgebildet ist, um basierend auf der Lage der zumindest einen Seitenbegrenzung (110) Sicherheitsinformation über eine Konfiguration der Patientenlagerungsvorrichtung (100) zu bestimmen und wobei ferner die Bestimmungseinrichtung (16) ausgebildet ist, um basierend auf der Sicherheitsinformation eine Alarmierungsinformation auszugeben, wenn die Konfiguration der Patientenlagerungsvorrichtung (100) einen Schwellenwert für ein Sicherheitsniveau unterschreitet, wobei das Sicherheitsniveau ein Maß einer mittleren Höhe der Seitenbegrenzung (110) über einer Liegefläche über dem zumindest einen Teilsegment (120) darstellt.

2. Vorrichtung (10) gemäß Anspruch 1, die ferner eine Schnittstelle (12) zur Ausgabe einer Information über die Lage der zumindest einen Seitenbegrenzung (110) aufweist.

3. Vorrichtung (10) gemäß einem der Ansprüche 1 oder 2, die eine Erfassungseinrichtung (14) zur Erfassung der optischen Bilddaten der Patientenlagerungsvorrichtung (100) umfasst, wobei die Erfassungseinrichtung (14) einen oder mehrere Sensoren aufweist, der/die zur Erfassung einer drei-dimensionalen Punktewolke als Bilddaten ausgebildet ist/sind.

4. Vorrichtung (10) gemäß einem der vorangehenden Ansprüche, wobei die Bestimmungseinrichtung (16) ferner ausgebildet ist, um basierend auf der Lage des zumindest einen Teilsegmentes (120) der Patientenlagerungsvorrichtung (100) eine Größe und eine Position einer Liegefläche der Patientenlagerungsvorrichtung (100) zu bestimmen.

**5.** Vorrichtung (10) gemäß Anspruch 4, wobei die Bestimmungseinrichtung (16) ausgebildet ist, um ferner zwei Längsseiten der Patientenlagerungsvorrichtung (100) zu bestimmen, und basierend auf den Längsseiten aus den Bilddaten Bildpunkte zumindest teilweise auszuschließen, die nicht zu der zumindest einen Seitenbegrenzung (110) der Patientenlagerungsvorrichtung (100) gehören.

**6.** Vorrichtung (10) gemäß Anspruch 5, wobei die Bestimmungseinrichtung (16) ausgebildet ist, um die Bilddaten ferner auf Bildpunkte einer Längsseite der Patientenlagerungsvorrichtung (100) einzuschränken.

**7.** Vorrichtung (10) gemäß Anspruch 6, wobei die Bestimmungseinrichtung (16) ausgebildet ist, um die Bilddaten auf eine Seitenebene der Patientenlagerungsvorrichtung (100) zu projizieren und um ein Projektionsbild zu erhalten.

**8.** Vorrichtung (10) gemäß Anspruch 7, wobei die Bestimmungseinrichtung (16) ausgebildet ist, um in dem Projektionsbild mittels einer Objekt-Detektion Seitenbegrenzungskandidaten zu bestimmen.

**9.** Vorrichtung (10) gemäß Anspruch 8, wobei die Bestimmungseinrichtung (16) ausgebildet ist, um die Seitenbegrenzungskandidaten zu bewerten und um basierend auf den bewerteten Seitenbegrenzungskandidaten die Lage der zumindest einen Seitenbegrenzung (110) zu bestimmen.

**10.** Vorrichtung (10) gemäß Anspruch 1, wobei die Bestimmungseinrichtung (16) ausgebildet ist, um die Sicherheitsinformation über eine Speichereinrichtung zu dokumentieren.

**11.** Vorrichtung (10) gemäß einem der Ansprüche 1 oder 10, wobei die Bestimmungseinrichtung (16) ausgebildet ist, um die Sicherheitsinformation über eine Darstellungseinrichtung auszugeben.

**12.** Vorrichtung (10) gemäß Anspruch 1, wobei das Sicherheitsniveau der Patientenlagerungsvorrichtung (100) auf einer relativen Lage des zumindest einen Teilsegmentes (120) zu der zumindest einen Seitenbegrenzung (110) basiert.

**13.** Vorrichtung (10) gemäß einem der Ansprüche 1 oder 12, wobei das Sicherheitsniveau ein Maß dafür darstellt, wie hoch ein Risiko eines Herausfallens eines Patienten aus der Patientenlagerungsvorrichtung (100) bei der bestimmten Konfiguration der Patientenlagerungsvorrichtung (100) ist.

**14.** Vorrichtung (10) gemäß einem der vorangehenden Ansprüche, wobei die Bestimmungseinrichtung (16) ausgebildet ist, um die Lage von zumindest zwei Teilsegmenten (120a; 120b) der Patientenlagerungsvorrichtung (100) zu bestimmen.

**15.** Verfahren zur Erfassung von optischen Bilddaten einer Patientenlagerungsvorrichtung (100) und zur Bestimmung einer Lage von zumindest einer Seitenbegrenzung (110) der Patientenlagerungsvorrichtung (100) basierend auf den Bilddaten, mit folgenden Schritten Bestimmen (80a) einer Lage von zumindest einem Teilsegment (120) der Patientenlagerungsvorrichtung (100) basierend auf den Bilddaten;

Bestimmen (80b) der Lage der zumindest einen Seitenbegrenzung (110) der Patientenlagerungsvorrichtung (100) basierend auf der Lage des zumindest einen Teilsegmentes (120); Bestimmen einer Sicherheitsinformation über eine Konfiguration der Patientenlagerungsvorrichtung (100) basierend auf der Lage der zumindest einen Seitenbegrenzung (110); und Ausgeben einer Alarminformation basierend auf der Sicherheitsinformation, wenn die Konfiguration der Patientenlagerungsvorrichtung (100) einen Schwellenwert für ein Sicherheitsniveau unterschreitet, wobei das Sicherheitsniveau ein Maß einer mittleren Höhe der Seitenbegrenzung (110) über einer Liegefläche über dem zumindest einen Teilsegment (120) darstellt.

**16.** Programm mit einem Programmcode zur Durchführung des Verfahrens gemäß Anspruch 15, wenn der Programmcode auf einem Computer, einem Prozessor oder einer programmierbaren Hardwarekomponente ausgeführt wird.

**Claims**

**1.** Apparatus (10) for capturing optical image data of a patient supporting apparatus (100) and for determining a location of at least one lateral boundary (110) of the patient supporting apparatus (100) on the basis of the image data, wherein the apparatus (10) comprises a determining device (16) designed for firstly determining a location of at least one partial segment (120) of the patient supporting apparatus (100) on the basis of the image data, and wherein the apparatus (10) is designed to determine the location of the at least one lateral boundary (110) of the patient supporting apparatus (100) on the basis of the location of the at least one partial segment (120), and wherein the determining device (16) is designed to determine safety information about a configuration of the patient supporting apparatus (100) on the basis of the location of the at least one lateral boundary (100), and wherein furthermore the determining device (16) is designed to output alerting information on the basis of the safety information if the configuration of the patient supporting apparatus (100) falls below a threshold value for a safety level, wherein the safety level represents a measure of an average height of the lateral boundary (110) above a lying surface over the at least one partial segment (120).

2. Apparatus (10) according to Claim 1, which furthermore has an interface (12) for outputting information about the location of the at least one lateral boundary (110).

3. Apparatus (10) according to either of Claims 1 and 2, which comprises a capturing device (14) for capturing the optical image data of the patient supporting apparatus (100), wherein the capturing device (14) has one or more sensors designed for capturing a three-dimensional point cloud as image data.

4. Apparatus (10) according to any of the preceding claims, wherein the determining device (16) is furthermore designed to determine a size and a position of a lying surface of the patient supporting apparatus (100) on the basis of the location of the at least one partial segment (120) of the patient supporting apparatus (100).

5. Apparatus (10) according to Claim 4, wherein the determining device (16) is designed furthermore to determine two longitudinal sides of the patient supporting apparatus (100) and, on the basis of the longitudinal sides, at least partly to exclude from the image data image points which do not belong to the at least one lateral boundary (110) of the patient supporting apparatus (100).

6. Apparatus (10) according to Claim 5, wherein the determining device (16) is designed furthermore to restrict the image data to image points of one longitudinal side of the patient supporting apparatus (100).

7. Apparatus (10) according to Claim 6, wherein the determining device (16) is designed to project the image data onto a lateral plane of the patient supporting apparatus (100) and to obtain a projection image.

8. Apparatus (10) according to Claim 7, wherein the determining device (16) is designed to determine lateral boundary candidates in the projection image by means of an object detection.

9. Apparatus (10) according to Claim 8, wherein the determining device (16) is designed to assess the lateral boundary candidates and to determine the location of the at least one lateral boundary (110) on the basis of the assessed lateral boundary candidates.

10. Apparatus (10) according to Claim 1, wherein the determining device (16) is designed to document the safety information by way of a storage device.

11. Apparatus (10) according to either of Claims 1 and 10, wherein the determining device (16) is designed to output the safety information by way of a display device.

12. Apparatus (10) according to Claim 1, wherein the safety level of the patient supporting apparatus (100) is based on a relative location of the at least one partial segment (120) with respect to the at least one lateral boundary (110).

13. Apparatus (10) according to either of Claims 1 and 12, wherein the safety level represents a measure of how high is a risk of a patient falling out of the patient supporting apparatus (100) in the case of the determined configuration of the patient supporting apparatus (100).

14. Apparatus (10) according to any of the preceding claims, wherein the determining device (16) is designed to determine the location of at least two partial segments (120a; 120b) of the patient supporting apparatus (100).

15. Method for capturing optical image data of a patient supporting apparatus (100) and for determining a location of at least one lateral boundary (110) of the patient supporting apparatus (100) on the basis of the image data, comprising the following steps:

determining (80a) a location of at least one partial segment (120) of the patient supporting apparatus (100) on the basis of the image data; determining (80b) the location of the at least one lateral boundary (110) of the patient supporting apparatus (100) on the basis of the location of the at least one partial segment (120); determining safety information about a configuration of the patient supporting apparatus (100) on the basis of the location of the at least one lateral boundary (110); and outputting alarm information on the basis of the safety information if the configuration of the patient supporting apparatus (100) falls below a threshold value for a safety level, wherein the safety level represents a measure of an average height of the lateral boundary (110) above a lying surface over the at least one partial segment (120).

**16.** Program comprising a program code for carrying out the method according to Claim 15 when the program code is executed on a computer, a processor or a programmable hardware component.

## Revendications

**1.** Dispositif (10) dévolu à la capture de données d'images optiques d'un dispositif (100) de positionnement de patients et à la détermination, sur la base desdites données d'images, d'une position d'au moins une délimitation latérale (110) dudit dispositif (100) de positionnement de patients, sachant que ledit dispositif (10) comprenant un appareil de détermination (16) qui est conçu pour déterminer tout d'abord une position d'au moins un segment partiel (120) du dispositif (100) de positionnement de patients, sur la base des données d'images, et sachant que ledit dispositif (10) est conçu pour déterminer la position de la délimitation latérale (110) à présence minimale dudit dispositif (100) de positionnement de patients, sur la base de ladite position dudit segment partiel (120) à présence minimale, sachant que l'appareil de détermination (16) est conçu pour déterminer, sur la base de ladite position de la délimitation latérale (110) à présence minimale, une information de sécurité relative à une configuration du dispositif (100) de positionnement de patients et sachant, en outre, que ledit appareil de détermination (16) est conçu pour délivrer une information d'alerte, sur la base de ladite information de sécurité, lorsque ladite configuration du dispositif (100) de positionnement de patients dépasse négativement une valeur de seuil allouée à un niveau de sécurité, lequel niveau de sécurité représente un critère estimatif d'une hauteur moyenne de la délimitation latérale (110) au-dessus d'une surface de couchage surplombant le segment partiel (120) à présence minimale.

**2.** Dispositif (10) selon la revendication 1, par ailleurs muni d'un interface (12) affecté à la délivrance d'une information relative à la position de la délimitation latérale (110) à présence minimale.

**3.** Dispositif (10) selon l'une des revendications 1 ou 2, incluant un appareil détecteur (14) dédié à la capture des données d'images optiques du dispositif (100) de positionnement de patients, lequel appareil détecteur (14) est doté d'un ou de plusieurs capteur(s) conçu(s) pour détecter un nuage de points tridimensionnel en tant que données d'images.

**4.** Dispositif (10) selon l'une des revendications précédentes, l'appareil de détermination (16) est conçu en outre pour déterminer, sur la base de ladite position du segment partiel (120) à présence minimale du dispositif (100) de positionnement de patients, une taille et un emplacement d'une surface de couchage dudit dispositif (100) de positionnement de patients.

**5.** Dispositif (10) selon la revendication 4, l'appareil de détermination (16) étant conçu pour déterminer, par ailleurs, deux côtés longitudinaux du dispositif (100) de positionnement de patients et pour exclure au moins partiellement des données d'images, sur la base desdits côtés longitudinaux, des pixels ne faisant pas partie de la délimitation latérale (110) à présence minimale dudit dispositif (100) de positionnement de patients.

**6.** Dispositif (10) selon la revendication 5, l'appareil de détermination (16) étant conçu pour cantonner, en outre, les données d'images à des pixels d'un côté longitudinal du dispositif (100) de positionnement de patients.

**7.** Dispositif (10) selon la revendication 6, l'appareil de détermination (16) étant conçu pour projeter les données d'images sur un plan latéral du dispositif (100) de positionnement de patients, et pour obtenir une image de projection.

**8.** Dispositif (10) selon la revendication 7, l'appareil de détermination (16) étant conçu pour déterminer, au moyen d'une détection d'objets, des postulants de délimitations latérales dans l'image de projection.

**9.** Dispositif (10) selon la revendication 8, l'appareil de détermination (16) étant conçu pour évaluer les postulants de délimitations latérales et pour déterminer, sur la base des postulants de délimitations latérales évalués, la position de la délimitation latérale (110) à présence minimale.

**10.** Dispositif (10) selon la revendication 1, l'appareil de détermination (16) étant conçu pour documenter l'information de sécurité par l'intermédiaire d'un appareil de mémorisation.

**11.** Dispositif (10) selon l'une des revendications 1 ou 10, l'appareil de détermination (16) étant conçu pour délivrer l'information de sécurité par l'intermédiaire d'un appareil de visualisation.

**12.** Dispositif (10) selon la revendication 1, le niveau de sécurité du dispositif (100) de positionnement de patients étant fondé sur une position relative du segment partiel (120), à présence minimale, vis-à-vis de la délimitation latérale (110) à présence minimale.

**13.** Dispositif (10) selon l'une des revendications 1 ou 12, le niveau de sécurité constituant un critère estimatif de l'ampleur d'un risque de chute d'un patient, hors du dispositif (100) de positionnement de patients, en présence de la configuration déterminée dudit dispositif (100) de positionnement de patients.

**14.** Dispositif (10) selon l'une des revendications précédentes, l'appareil de détermination (16) étant conçu pour déterminer la position d'au moins deux segments partiels (120a ; 120b) du dispositif (100) de positionnement de patients.

**15.** Procédé conçu pour capturer des données d'images optiques d'un dispositif (100) de positionnement de patients et pour déterminer, sur la base desdites données d'images, une position d'au moins une délimitation latérale (110) dudit dispositif (100) de positionnement de patients, comprenant les étapes suivantes :

détermination (80a), sur la base des données d'images, d'une position d'au moins un segment partiel (120) du dispositif (100) de positionnement de patients ; détermination (80b), sur la base de ladite position du segment partiel (120) à présence minimale, de la position de la délimitation latérale (110) à présence minimale dudit dispositif (100) de positionnement de patients ;

détermination d'une information de sécurité par l'intermédiaire d'une configuration dudit dispositif (100) de positionnement de patients fondée sur ladite position de la délimitation latérale (110) à présence minimale ; et

délivrance d'une information d'alerte, sur la base de ladite information de sécurité, lorsque ladite configuration du dispositif (100) de positionnement de patients dépasse négativement une valeur de seuil allouée à un niveau de sécurité, lequel niveau de sécurité représente un critère estimatif d'une hauteur moyenne de la délimitation latérale (110) au-dessus d'une surface de couchage surplombant le segment partiel (120) à présence minimale.

**16.** Programme muni d'un code, dédié à la mise en oeuvre du procédé conforme à la revendication 15 lorsque ledit code du programme est exécuté sur un ordinateur, un processeur ou un composant de matériel programmable.

Fig. 1

Fig. 2

Fig. 3

```
                    ┌─────────────────────┐
                    │   Bestimmung von    │
                    │   Tiefeninformation │〜40a
                    └─────────────────────┘
                      │                 │
                      ▼                 ▼
              ┌──────────┐          ┌──────────┐
              │  Direkt  │〜40b      │ Indirekt │〜40c
              └──────────┘          └──────────┘
               │        │        40f   │          │
               ▼        ▼         〜    ▼          ▼
        ┌─────────┐ ┌──────────────┐ ┌──────────────┐ ┌─────────────────┐
        │ Laufzeit│ │(De-)Fokussie-│ │ Triangulation│ │  Oberflächen-   │
        │         │ │    rung      │ │              │ │  charakteristik │
        └─────────┘ └──────────────┘ └──────────────┘ └─────────────────┘
           〜           〜             │      │      │           〜
          40d          40e            ▼      ▼      ▼           40g
                            ┌──────────────┐ ┌──────────┐ ┌──────────┐
                            │ Strukturiertes│ │ Bewegung │ │  Stereo- │
                            │    Licht      │ │          │ │  kamera  │
                            └──────────────┘ └──────────┘ └──────────┘
                                 〜              〜           〜
                                40h             40i          40j
```

Fig. 4

Fig. 5

20

50a

Start

50b

Bestimmung der PLV-Segmente

50c

Bestimmung der PLV-Längsseiten

50d

Entfernen aller nicht relevanten Punkte

50e

Projizieren der Punkte auf Seitenebene

50f

Erstellen mind. eines Bildes aus der Projektion

50g

Objekt-Detektion

50h

Bewerten der Detektionen

50i

Beschreibe Seiten-begrenzungen

50j

Bestimme Sicher-heitsmaß für Bettkonfiguration

50k

Ende

Fig. 6

Fig. 7

Fig. 8

60a       60b       60c       60d

```
┌─────────┐     ┌──────────────┐     ┌──────────────┐     ┌──────────────┐
│  Start  │ ──> │  Berechne    │ ──> │  Überführe   │ ──> │  Berechne    │
│         │     │  PLV-        │     │  PLV-        │     │  Geraden im  │
└─────────┘     │  Histogramm  │     │  Histogramm  │     │  Bild        │
                └──────────────┘     │  in Bild     │     └──────────────┘
                                     └──────────────┘            │
                                                                 ▼
┌─────────┐     ┌──────────────┐     ┌──────────────┐     ┌──────────────┐
│  Ende   │ <── │  Wähle       │ <── │  Bestimme    │ <── │  Gruppiere   │
│         │     │  bestes      │     │  mittlere    │     │  Geraden     │
└─────────┘     │  Geradenpaar │     │  Gerade pro  │     │              │
                └──────────────┘     │  Gruppe      │     └──────────────┘
                                     └──────────────┘
```

60h       60g       60f       60e

Fig. 9

Fig. 10

Fig. 11

Fig. 12

Fig. 13

Fig. 14

Fig. 15

Fig. 16

70a

Unterteile
Liegefläche
in Teilabschnitte

70b

Bestimme Höhe
über Bettgitter-
Oberkante pro
Abschnitt

v

70c

Bestimme
mittlere Höhe
über alle Abschnitte

a

70d

Vergleiche m
mit
Schwellwert S

70e

m<S

ja → 70f Gefä...

nein → 70g ungefährlich

70h

Unterteile
Liegefläche
in Teilabschnitte

70i

Bestimme Höhe
über Bettgitter-
Oberkante pro
Abschnitt

$v_1,... v_L$

70j

Bestimme
mittlere Höhe
pro
Lagesegment

$m_1,... m_L$

70k

Vergleiche mit
individuellen
Schwellwerten
$S_1,..., S_L$

70l

Anzahl oder Anteil
überschritten?

ja → Gefährlich 70m

nein → ungefährlich 70n

70o

Unterteile
Liegefläche
in Teilabschnitte

70p

Bestimme Höhe
über Bettgitter-
Oberkante pro
Abschnitt

v

70q

Bestimme
Anteil der vi
größer
Schwellwert S

a

70r

Vergleiche a
mit zweitem
Schwellwert
$S_2$

70s

a>S2 ?

ja → Gefährlich 70t

nein → ungefährlich 70u

Fig. 17

Fig. 18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120075464 A1

- DE 102015013031

**In der Beschreibung aufgeführte Nicht-Patentliteratur**

- **BESL, P. J.** Method for registration of 3-D shapes. *Robotics-DL tentative,* 1992, 586-606
- **FISCHLER, M. A.** Random sample consensus: a paradigm for model fitting with applications to image analysis and automated cartography. *Communications of the ACM,* 1981, 381-395
- **HARTMAN, F.** Robotersteuerung durch Gesten. Masterarbeit Universität zu Lübeck, 2011
- **KONG, T. ; ROSENFELD, A.** Topological Algorithms for Digital Image Processing. Elsevier Science, Inc, 1996
- **SHAPIRO, L. ; STOCKMAN, G.** Computer Vision. Prentice-Hall, 2001
- **BRON, C. ; KERBOSCH, J.** *Algorithm 457: finding all cliques of an undirected graph, Communications of the ACM,* 1973, 575-577
- **CANNY, J.** A Computational Approach to Edge Detection. *IEEE Transactions on Pattern Analysis and Machine Intelligence,* 1986
- **CAPEZUTI, E. ; WAGNER, L. ; BRUSH, B.** Consequences of an intervention to reduce restrictive side rail use in nursing homes. *Journal of the American Geriatrics Society,* 2007, 334-341
- **DALAL, N. ; TRIGGS, B.** Histograms of Oriented Gradients for Human Detection. *IEEE Computer Society Conference on Computer Vision and Pattern Recognition,* 2005
- **DUDA, R. ; HART, P. E.** Use ofthe Hough Transformation to Detect Lines and Curves in Pictures. *Comm. ACM,* 1972, 11-15

- **HARTMAN, F.** Robotersteuerung durch Gesten. Universität Lübeck, 2011
- **JOLLIFFE, I. T.** Principal Component Analysis. Springer, 2002
- **SOILLE, P.** Morphological Image Analysis: Principles and Applications. Springer-Verlag, 1999
- **TALERICO, K. A. ; CAPEZUTI, E.** Myths and Facts About Side Rails: Despite ongoing debates about safety and efficacy, side rails are still a standard component of care in many hospitals, so how do you determine their safe use?. *AJN The American Journal of Nursing,* 2001, 43-48
- **VIOLA, P. ; JONES, M.** Rapid object detection using a boosted cascade of simple features. *CONFERENCE ON COMPUTER VISION AND PATTERN RECOGNITION,* 2001
- **OJALA, T. ; M. PIETIKAINEN ; T. MAENPAA.** Multiresolution Gray-scale and Rotation Invariant Texture Classification With Local Binary Patterns. *IEEE Transactions on Pattern Analysis and Machine Intelligence.,* Juli 2002, vol. 24 (7), 971-987
- **VIOLA, P. ; M. J. JONES.** Rapid Object Detection using a Boosted Cascade of Simple Features. *Proceedings of the 2001 IEEE Computer Society Conference,* 15. April 2001, vol. 1, I-511-I-518
- **CORTES, CORINNA ; VLADIMIR VAPNIK.** Support-vector networks. *Machine learning,* 1995, vol. 20 (3), 273-297
- **BREIMAN L.** Random forests. *Machine Learning,* 2001, 5-32